# EUROPEAN PATENT SPECIFICATION

(11) **EP 3 315 138 B1**
(45) Date of publication and mention of the grant of the patent: **27.05.2020**
(21) Application number: 16814244.6
(22) Date of filing: 15.06.2016
(51) Int. Cl.: A61K 47/42, A61K 8/34, A61K 8/64, A61K 8/86, A61K 9/06, A61K 31/167, A61K 31/506, A61K 47/10, A61K 47/12, A61K 47/14, A61K 47/24, A61Q 1/00, A61Q 19/00

(54) **STICK-SHAPED SUBSTRATE CONTAINING LIPID-PEPTIDE-TYPE COMPOUND**
STABFÖRMIGES SUBSTRAT MIT LIPID-PEPTID-ARTIGER VERBINDUNG
MATÉRIAU DE BASE EN BÂTONNET COMPRENANT UN COMPOSÉ PEPTIDE LIPIDIQUE

(30) Priority: 24.06.2015 JP 2015127107
(43) Date of publication of application: 02.05.2018
(73) Proprietor: Nissan Chemical Corporation, Tokyo (JP)
(72) Inventor: KASHINO, Tsubasa, Funabashi-shi Chiba 274-0052 (JP); IMOTO, Takayuki, Funabashi-shi Chiba 274-0052 (JP)
(74) Representative: Hoffmann Eitle
(86) International application number: PCT/JP2016/067828
(87) International publication number: WO 2016/208473

(56) References cited:
- EP-A1- 3 210 613
- WO-A1-2010/049389
- WO-A1-2010/049390
- WO-A1-2011/052613
- WO-A1-2014/003015
- WO-A1-2014/054702
- WO-A1-2015/099074
- JP-A- 2015 051 961

## Description

### TECHNICAL FIELD

The present invention relates to the use of at least one saturated or unsaturated monohydric alcohol having a carbon atom number of 8 to 30 in a stick shaped solid base material for external use on skin comprising a lipid peptide compound, and having excellent high-temperature stability.

### BACKGROUND ART

Aqueous solid compositions have been marketed or proposed as various products for cosmetics and the like, because they give a highly refreshing feel upon application to the skin or the like, and give a light feel upon use without leaving stickiness after use, as compared to oleaginous solid compositions.

Conventionally proposed aqueous solid compositions include an oil-in-water solid makeup cosmetic preparation comprising water, a fatty acid soap, an oil, and powder (Patent Document 1); and a stick-shaped aqueous cosmetic preparation comprising an alkyl and/or alkenyl oligoglycoside, an oily substance, and a nonionic emulsifier (Patent Document 2).

Aqueous gel compositions are also one example of such aqueous solid compositions. Various compounds such as a polymer gelator and a low-molecular-weight gelator have been proposed as additives for producing such aqueous gels. For example, a low-molecular-weight lipid peptide gelator that has high biological safety and is expected to be applied to pharmaceutical materials or the like has been recently proposed.

Furthermore, Patent Document 3 describes an external use composition for anti-aging, comprising (A) a specific lipopeptide compound, or a pharmaceutically acceptable salt thereof.

Patent Document 4 relates to compositions which are suitable for relaxing the muscles and thus for substantially reducing facial expression wrinkles. Said compositions contain at least one DNA repair enzyme and at least one oligopeptide which can have an N-C₂-C₂₄ acyl group and/or can be esterified, and additionally, based on the respective weight of the composition, 0.001 to 2% by weight of at least one apple core extract, and 0.001 to 2% by weight of hyaluronic acid in a cosmetically acceptable carrier.

Similarly, Patent Document 5 describes compositions which are suitable for substantially reducing wrinkles in three dimensions. Said compositions contain at least one DNA repair enzyme and at least one oligopeptide which can have an N-C₂-C₂₄ acyl group and/or can be esterified, and additionally, based on the respective weight of the composition, 0.001 to 1% by weight of at least one hydroxystilbene oligomer, the alkyl ethers thereof and/or the esters thereof, 0.001 to 2% by weight of at least one apple core extract, and at least one oil-soluble UV filter which comprises at least one alkyl-substituted and/or alkxoy-substituted dibenzyolmethane derivative, in a cosmetically acceptable carrier.

### Prior Art Documents

### Patent Documents

Patent Document 1: Japanese Patent Application Publication No. H3-279319 (JP H3-279319 A)
Patent Document 2: Japanese Patent Application Publication (Translation of PCT Application) No. 2002-516818 (JP 2002-516818 A)
Patent Document 3: EP 3 210 613 A1
Patent Document 4: WO 2010/049389 A1
Patent Document 5: WO 2010/049390 A1

### SUMMARY OF THE INVENTION

### Problem to be Solved by the Invention

An aqueous gel obtained using the above-mentioned low-molecular-weight lipid peptide gelator has a relatively low breaking strength, which makes it difficult to apply the aqueous gel to products for applications requiring a certain level of strength, such as a stick-shaped solid base material for external use on skin. Moreover, it is expected that a solid base material for external use on skin, when in use, is often kept under high-temperature conditions at 50°C or higher, for example, in a car in the middle of summer. However, for example, the aqueous gel obtained using the above-mentioned low-molecular-weight lipid peptide gelator cannot remain in the solid state in such a high-temperature environment, possibly leading to a loss of the product performance or appearance. Thus, it has been an important issue to ensure the stability of the base material to temperature (heat).

In view of the above-described circumstances, a problem to be solved by the present invention is to provide a solid base material for external use on skin that has a breaking strength sufficiently high to be usable as a stick-shaped base material or the like, and has excellent thermal stability.

### Means for Solving the Problem

As a result of diligent study to solve the aforementioned problem, the present inventors have found that, in the formation of a hydrogel using water and a lipid peptide compound (gelator) comprising a low-molecular-weight lipid peptide or a pharmaceutically usable salt thereof, a suitable amount of a higher monohydric alcohol can be added to produce a gel having dramatically improved thermal stability that can be suitably used as a solid base material for external use on skin, particularly a stick-shaped base material, thus completing the present invention.

In summary, a first aspect of the present invention relates to the use of at least one saturated or unsaturated monohydric alcohol having a carbon atom number of 8 to 30 in a stick shaped solid base material for external use on skin comprising:
a surfactant and water;
a lipid peptide compound comprising at least one of compounds of formulas (1) to (3): 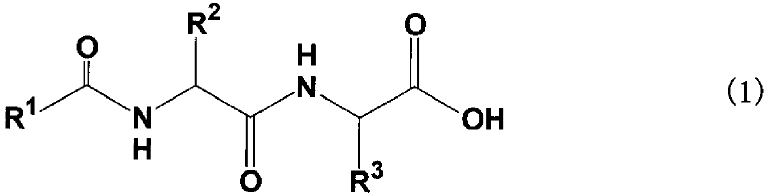 (wherein R¹ is a C₉₋₂₃ aliphatic group; R² is a hydrogen atom or a C₁₋₄ alkyl group optionally having a C₁ or C₂ branched chain; and R³ is a -(CH₂)ₙ-X group, wherein n is a number from 1 to 4, and X is amino group, guanidino group, -CONH₂ group, or a 5-membered ring or 6-membered ring optionally having one to three nitrogen atoms or a fused heterocyclic ring composed of the 5-membered ring and the 6-membered ring); 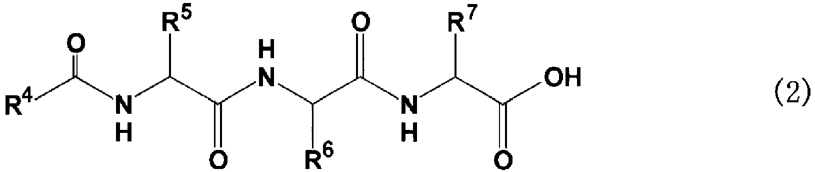 (wherein R⁴ is a C₉₋₂₃ aliphatic group; and R⁵ to R⁷ are each independently a hydrogen atom, a C₁₋₄ alkyl group optionally having a C₁ or C₂ branched chain, or a -(CH₂)ₙ-X group, wherein n is a number from 1 to 4, and X is amino group, guanidino group, -CONH₂ group, or a 5-membered ring or 6-membered ring optionally having one to three nitrogen atoms or a fused heterocyclic ring composed of the 5-membered ring and the 6-membered ring); 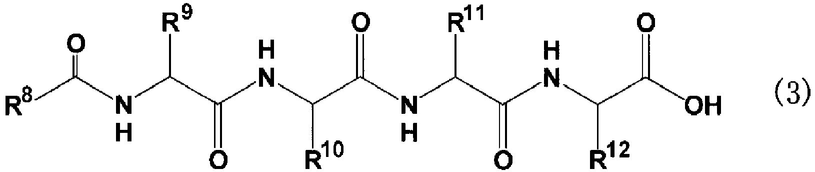 (wherein R⁸ is a C₉₋₂₃ aliphatic group; and R⁹ to R¹² are each independently a hydrogen atom, a C₁₋₄ alkyl group optionally having a C₁ or C₂ branched chain, or a -(CH₂)ₙ-X group, wherein n is a number from 1 to 4, and X is amino group, guanidino group, -CONH₂ group, or a 5-membered ring or 6-membered ring optionally having one to three nitrogen atoms or a fused heterocyclic ring composed of the 5-membered ring and the 6-membered ring); or
pharmacologically usable salts thereof for increasing the thermal stability of the stick shaped solid base material.

A second aspect of the present invention relates to the use according to the first aspect, wherein the solid base material further comprises a 1,2-alkanediol or 1,3-alkanediol.

A third aspect of the present invention relates to the use according to the first or second aspect, wherein the solid base material further comprises at least one fatty acid.

A fourth aspect of the present invention relates to the use according to the second or third aspect, wherein the solid base material further comprises at least one polyhydric alcohol different from the 1,2-alkanediol or 1,3-alkanediol.

A fifth aspect of the present invention relates to the use according to any one of the first to fourth aspects, wherein the surfactant is one or more compounds selected from the group consisting of ethylene glycol alkyl ethers, phospholipids, polyglycerin fatty acid esters, and polyoxyethylene polyoxypropylene alkyl ethers.

A sixth aspect of the present invention relates to the use according to any one of the third to fifth aspects, wherein the fatty acid is stearic acid.

A seventh aspect of the present invention relates to the use according to any one of the fourth to sixth aspects, wherein the polyhydric alcohol is glycerin, propylene glycol, or polyethylene glycol.

An eighth aspect of the present invention relates to the use according to any one of the first to seventh aspects, wherein the solid base material further comprises at least one oleaginous base.

A ninth aspect of the present invention relates to the use according to any one of the first to eighth aspects, wherein the solid base material further comprises at least one organic acid.

A tenth aspect of the present invention relates to the use according to any one of the first to ninth aspects, wherein the solid base material is used for cosmetics or pharmaceuticals.

An eleventhaspect of the present invention relates to a method for producing the solid base material for external use on skin as defined in the first aspect, comprising the steps of:
a heating step of heating a mixture system to a temperature not lower than room temperature and lower than 100°C, wherein the mixture system comprises a surfactant and water, and a lipid peptide compound comprising at least one of compounds of formulas (1) to (3) or pharmacologically usable salts thereof;
a preparation step of adding at least one saturated or unsaturated monohydric alcohol having a carbon atom number of 8 to 30 to the heated mixture system to prepare the aqueous composition according to any one of the twelfth to seventeenth aspects;
a mixing step of adding the prepared aqueous composition to an aqueous phase heated to a temperature not lower than room temperature and lower than 100°C, followed by mixing, or adding an aqueous phase heated to a temperature not lower than room temperature and lower than 100°C to the prepared aqueous composition, followed by mixing; and
a cooling step of cooling the mixture obtained in the mixing step to form a gel.

A twelfth aspect of the present invention relates to the method according to the eleventh aspect, wherein in the mixing step, a solution of a drug is further added.

### Effects of the Invention

The present invention provides the use of at least one saturated or unsaturated monohydric alcohol having a carbon atom number of 8 to 30 in a stick shaped solid base material for external use on skin having excellent thermal stability. The present invention also achieves an additional effect in that a gel having a breaking strength dramatically higher than that of conventional gels is obtained by adding a 1,2-alkanediol or 1,3-alkanediol as a solubilizer for the lipid peptide compound, and further adding a surfactant. A preferred embodiment of the present invention provides the use of at least one saturated or unsaturated monohydric alcohol having a carbon atom number of 8 to 30 in a stick shaped solid base material for external use on skin that has a strength higher than that of conventional aqueous gel base materials, and is weakly acidic at around pH 5.

The present invention further achieves an effect in that a gel base material is provided that can exhibit a high water content of approximately 90% by mass, and can contain an oleaginous ingredient simultaneously.

The lipid peptide compound contained in the solid base material for external use on skin is a highly safe artificial low-molecular-weight compound that is composed of a lipid and a peptide only. Moreover, for example, the lipid peptide compound allows an aqueous gel to be formed without using a crosslinking agent or the like that is required to form a conventionally proposed synthetic polymer gel. As a result, no unreacted matter such as unreacted crosslinking agent remains in the resulting solid base material for external use on skin.

Furthermore, the main ingredients contained as additives in the solid base material for external use on skin are versatile additives for foodstuffs, cosmetics, and pharmaceuticals.

That is, the solid base material for external use on skin has a high level of biological safety, and hence, is extremely useful for the above-described uses, particularly from the viewpoint of a high level of safety required in materials for medicinal use or materials for cosmetic use, for example.

Furthermore, the solid base material for external use on skin is expected to serve as a base material that gives a refreshing feel, is neither broken nor deformed, and spreads well when applied to human skin or the like, and thus, is extremely useful as a base material for cosmetics or pharmaceuticals, and particularly as a stick-shaped base material.

Described is also an aqueous composition suitable as a premix raw material for the solid base material for external use on skin.

Through the use of the aqueous composition, a solid base material for external use on skin in the form of a gel can be provided that has a strength required for a stick-shaped base material, particularly even when it contains a large amount of an organic acid such as ascorbic acid.

Furthermore, through the use of a suitable amount of an alkyl alcohol, the present invention provides a solid base material that has excellent storage stability, and can favorably maintain the solid state even under high-temperature conditions that may occur in daily life.

### MODES FOR CARRYING OUT THE INVENTION

The present invention relates to the use of at least one saturated or unsaturated monohydric alcohol having a carbon atom number of 8 to 30 (hereinafter also referred to as an alkyl alcohol) in a stick shaped solid base material for external use on skin comprising a surfactant and water, a lipid peptide compound comprising at least one of compounds of formulas (1) to (3) or pharmacologically usable salts thereof, for increasing the thermal stability of the stick shaped solid base material, the solid base material further optionally comprising a 1,2-alkanediol or 1,3-alkanediol, a fatty acid, a polymer compound, an oleaginous base, an organic acid, and other additives.

Described is also an aqueous composition comprising the surfactant, water, the lipid peptide compound, and an alkyl alcohol as a thermal stabilizer, the aqueous composition further optionally comprising a 1,2-alkanediol or 1,3-alkanediol, a fatty acid, a polymer compound, an oleaginous base, an organic acid, and other additives.

The solid base material for external use on skin is suitable for cosmetics or pharmaceuticals, and can be used particularly as a stick-shaped base material. As used herein, the "stick-shaped base material" refers to a bar-shaped base material having a strength sufficiently high to maintain the bar shape and to be applicable to the skin or the like (i.e., capable of maintaining the shape upon application). The strength required in the stick-shaped base material is, for example, a breaking strength of 0.4 × 10⁵ to 8.0 × 10⁵ Pa, preferably 1.0 × 10⁵ to 7.0 × 10⁵ Pa, and more preferably 1.0 × 10⁵ to 6.0 × 10⁵ Pa.

In particular, the solid base material for external use on skin can remain in the solid state under high-temperature conditions at 50°C or higher that are expected to occur in instances of actual use and storage to which the solid base material may be applied. That is, the solid base material for external use on skin can stably remain in the solid state under storage conditions at 50°C, which serve as a criterion of thermal stability.

Each of the components will be described hereinafter.

### [Lipid Peptide Compound]

As the lipid peptide compound to be used in the solid base material for external use on skin or the aqueous composition, the compounds (lipid peptides) of formulas (1) to (3) or pharmacologically usable salts thereof (low-molecular-weight compounds each having a lipid moiety as a hydrophobic moiety and a peptide moiety as a hydrophilic moiety) can be used.

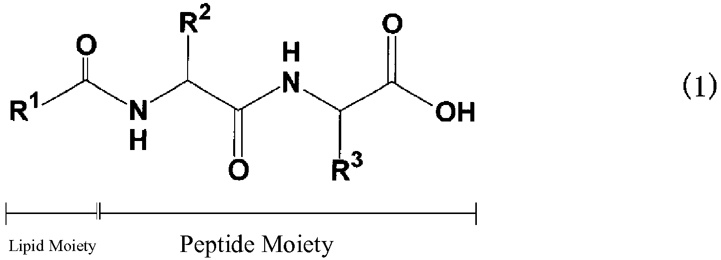

In formula (1), R¹ is a C₉₋₂₃ aliphatic group, and preferably a linear aliphatic group having a carbon atom number of 11 to 23, and optionally having zero to two unsaturated bonds.

Specific examples of the lipid moiety (acyl group) composed of R¹ and the adjacent carbonyl group include lauroyl group, dodecylcarbonyl group, myristoyl group, tetradecylcarbonyl group, palmitoyl group, margaroyl group, oleoyl group, elaidoyl group, linoleoyl group, stearoyl group, vaccenoyl group, octadecylcarbonyl group, arachidoyl group, eicosylcarbonyl group, behenoyl group, elkanoyl group, docosylcarbonyl group, lignoceroyl group, and nervonoyl group; and particularly preferred examples include lauroyl group, myristoyl group, palmitoyl group, margaroyl group, stearoyl group, oleoyl group, elaidoyl group, and behenoyl group.

In formula (1), R² included in the peptide moiety is a hydrogen atom or a C₁₋₄ alkyl group optionally having a C₁ or C₂ branched chain.

The C₁₋₄ alkyl group optionally having a C₁ or C₂ branched chain refers to an alkyl group that has a C₁₋₄ main chain, and optionally has a C₁ or C₂ branched chain. Specific examples of the C₁₋₄ alkyl group include methyl group, ethyl group, n-propyl group, i-propyl group, n-butyl group, i-butyl group, sec-butyl group, and tert-butyl group.

R² is preferably a hydrogen atom or a C₁₋₃ alkyl group optionally having a C₁ branched chain, and is more preferably a hydrogen atom.

The C₁₋₃ alkyl group optionally having a C₁ branched chain refers to an alkyl group that has a C₁₋₃ main chain, and optionally has a C₁ branched chain. Specific examples of the C₁₋₃ alkyl group include methyl group, ethyl group, n-propyl group, i-propyl group, i-butyl group, and sec-butyl group, with methyl group, i-propyl group, i-butyl group, and sec-butyl group being preferred.

In formula (1), R³ is a -(CH₂)ₙ-X group. In the -(CH₂)ₙ-X group, n is a number from 1 to 4; and X is amino group, guanidino group, -CONH₂ group, or a 5-membered ring group or 6-membered ring group optionally having one to three nitrogen atoms or a fused heterocyclic group composed of the 5-membered ring and the 6-membered ring. In the-(CH₂)ₙ-X group of R³, X is preferably amino group, guanidino group, carbamoyl group (-CONH₂ group), pyrrole group, imidazole group, pyrazole group, or indole group, and more preferably imidazole group. Furthermore, in the -(CH₂)ₙ-X group, n is preferably 1 or 2, and more preferably 1.

Thus, the -(CH₂)ₙ-X group is preferably aminomethyl group, 2-aminoethyl group, 3-aminopropyl group, 4-aminobutyl group, carbamoylmethyl group, 2-carbamoylethyl group, 3-carbamoylbutyl group, 2-guanidinoethyl group, 3-guanidinobutyl group, pyrrolemethyl group, 4-imidazolemethyl group, pyrazolemethyl group, or 3-indolemethyl group; more preferably 4-aminobutyl group, carbamoylmethyl group, 2-carbamoylethyl group, 3-guanidinobutyl group, 4-imidazolemethyl group, or 3-indolemethyl group; and still more preferably 4-imidazolemethyl group.

Particularly suitable examples of the lipid peptide compound of formula (1) include the following compounds each formed of a lipid moiety and a peptide moiety (amino acid assembly), wherein the amino acid abbreviations are alanine (Ala), asparagine (Asn), glutamine (Gln), glycine (Gly), histidine (His), isoleucine (Ile), leucine (Leu), lysine (Lys), tryptophan (Trp), and valine (Val): lauroyl-Gly-His, lauroyl-Gly-Gln, lauroyl-Gly-Asn, lauroyl-Gly-Trp, lauroyl-Gly-Lys, lauroyl-Ala-His, lauroyl-Ala-Gln, lauroyl-Ala-Asn, lauroyl-Ala-Trp, lauroyl-Ala-Lys; myristoyl-Gly-His, myristoyl-Gly-Gln, myristoyl-Gly-Asn, myristoyl-Gly-Trp, myristoyl-Gly-Lys, myristoyl-Ala-His, myristoyl-Ala-Gln, myristoyl-Ala-Asn, myristoyl-Ala-Trp, myristoyl-Ala-Lys; palmitoyl-Gly-His, palmitoyl-Gly-Gln, palmitoyl-Gly-Asn, palmitoyl-Gly-Trp, palmitoyl-Gly-Lys, palmitoyl-Ala-His, palmitoyl-Ala-Gln, palmitoyl-Ala-Asn, palmitoyl-Ala-Trp, palmitoyl-Ala-Lys; stearoyl-Gly-His, stearoyl-Gly-Gln, stearoyl-Gly-Asn, stearoyl-Gly-Trp, stearoyl-Gly-Lys, stearoyl-Ala-His, stearoyl-Ala-Gln, stearoyl-Ala-Asn, stearoyl-Ala-Trp, and stearoyl-Ala-Lys.

The most preferred examples of the lipid peptide compound of formula (1) include lauroyl-Gly-His, lauroyl-Ala-His-myristoyl-Gly-His, myristoyl-Ala-His; palmitoyl-Gly-His, palmitoyl-Ala-His; stearoyl-Gly-His, and stearoyl-Ala-His.

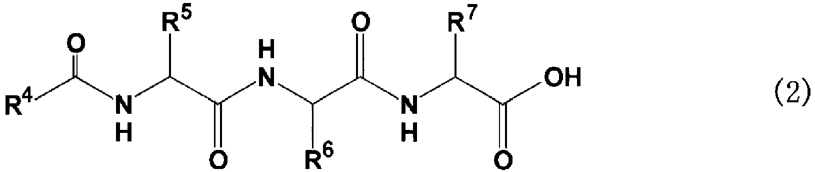

In formula (2), R⁴ is a C₉₋₂₃ aliphatic group, and preferred specific examples of R⁴ include the same groups as those defined by R¹ above.

In formula (2), R⁵ to R⁷ are each independently a hydrogen atom, a C₁₋₄ alkyl group optionally having a C₁ or C₂ branched chain, or a -(CH₂)ₙ-X group, and at least one of R⁵ to R⁷ is a -(CH₂)ₙ-X group. In formula (2), n is a number from 1 to 4, and X is amino group, guanidino group, -CONH₂ group, or a 5-membered ring group or 6-membered ring group optionally having one to three nitrogen atoms or a fused heterocyclic group composed of the 5-membered ring and the 6-membered ring. Preferred specific examples of R⁵ to R⁷ include the same groups as those defined by R² and R³ above.

Suitable examples of the lipid peptide compound of formula (2) include the following compounds each formed of a lipid moiety and a peptide moiety (amino acid assembly): myristoyl-Gly-Gly-His, myristoyl-Gly-Gly-Gln, myristoyl-Gly-Gly-Asn, myristoyl-Gly-Gly-Trp, myristoyl-Gly-Gly-Lys, myristoyl-Gly-Ala-His, myristoyl-Gly-Ala-Gln, myristoyl-Gly-Ala-Asn, myristoyl-Gly-Ala-Trp, myristoyl-Gly-Ala-Lys, myristoyl-Ala-Gly-His, myristoyl-Ala-Gly-Gln, myristoyl-Ala-Gly-Asn, myristoyl-Ala-Gly-Trp, myristoyl-Ala-Gly-Lys, myristoyl-Gly-His-Gly, myristoyl-His-Gly-Gly, palmitoyl-Gly-Gly-His, palmitoyl-Gly-Gly-Gln, palmitoyl-Gly-Gly-Asn, palmitoyl-Gly-Gly-Trp, palmitoyl-Gly-Gly-Lys, palmitoyl-Gly-Ala-His, palmitoyl-Gly-Ala-Gln, palmitoyl-Gly-Ala-Asn, palmitoyl-Gly-Ala-Trp, palmitoyl-Gly-Ala-Lys, palmitoyl-Ala-Gly-His, palmitoyl-Ala-Gly-Gln, palmitoyl-Ala-Gly-Asn, palmitoyl-Ala-Gly-Trp, palmitoyl-Ala-Gly-Lys, palmitoyl-Gly-His-Gly, and palmitoyl-His-Gly-Gly.

The most preferred examples of the above include lauroyl-Gly-Gly-His, myristoyl-Gly-Gly-His, palmitoyl-Gly-Gly-His, palmitoyl-Gly-His-Gly, palmitoyl-His-Gly-Gly, and stearoyl-Gly-Gly-His.

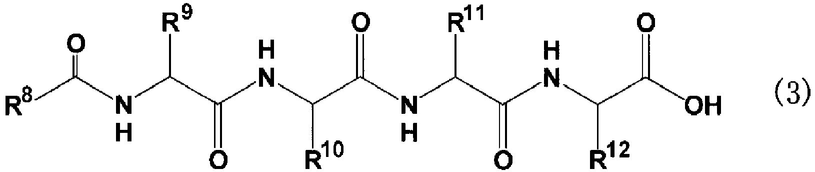

In formula (3), R⁸ is a C₉₋₂₃ aliphatic group, and preferred specific examples of R⁸ include the same groups as those defined by R¹ above.

In formula (3), R⁹ to R¹² are each independently a hydrogen atom, a C₁₋₄ alkyl group optionally having a C₁ or C₂ branched chain, or a -(CH₂)ₙ-X group, and at least one of R⁹ to R¹² is a -(CH₂)ₙ-X group. In formula (3), n is a number from 1 to 4, and X is amino group, guanidino group, -CONH₂ group, or a 5-membered ring group or 6-membered ring group optionally having one to three nitrogen atoms or a fused heterocyclic group composed of the 5-membered ring and the 6-membered ring. Preferred specific examples of R⁹ to R¹² include the same groups as those defined by R² and R³ above.

Thus, particularly suitable examples of the lipid peptide compound of formula (3) include lauroyl-Gly-Gly-Gly-His, myristoyl-Gly-Gly-Gly-His, palmitoyl-Gly-Gly-Gly-His, palmitoyl-Gly-Gly-His-Gly, palmitoyl-Gly-His-Gly-Gly, palmitoyl-His-Gly-Gly-Gly, and stearoyl-Gly-Gly-Gly-His.

In the present invention, the amount of the lipid peptide compound to be contained is, for example, 1 to 20% by mass, preferably 1 to 10% by mass, and more preferably 3 to 7% by mass, based on the total mass of the obtained solid base material for external use on skin.

In the present invention, the amount of the lipid peptide compound to be contained is, for example, 5 to 40% by mass, and preferably 10 to 30% by mass, based on the total mass of the obtained aqueous composition.

The lipid peptide compound to be used in the present invention comprises at least one of compounds (lipid peptides) of formulas (1) to (3) or pharmacologically usable salts thereof. These compounds may be used alone or in combination of two or more as a hydrogelator.

### [Surfactant]

As the surfactant to be used in the solid base material for external use on skin or the aqueous composition, a compound having a hydrophobic moiety and a hydrophilic moiety with a betaine structure in the molecule (hereinafter also referred to as a betaine compound), an ethylene glycol alkyl ether, a polyglycerin fatty acid ester, or a polyoxyethylene polyoxypropylene alkyl ether can be suitably used.

Examples of the betaine compound include betaine compounds known as amphoteric surfactants, for example, N-alkyl-N,N-dimethylamino acid betaines such as lauryl dimethylaminoacetic acid betaine (lauryl betaine); fatty acid amido alkyl-N,N-dimethylamino acid betaines such as cocamidopropyl betaine and lauramidopropyl betaine; imidazoline betaines such as sodium cocoamphoacetate and sodium lauroamphoacetate; alkyl sulfobetaines such as lauryl hydroxysulfobetaine and alkyl dimethyltaurine; betaine sulfates such as alkyl dimethylaminoethanol sulfate; and betaine phosphates such as alkyl dimethylaminoethanol phosphates.

Examples of the betaine compound further include glycerophospholipids such as phosphatidylcholine, phosphatidylethanolamine, phosphatidylserine, phosphatidylinositol, phosphatidylglycerol, diphosphatidylglycerol (cardiolipin), and phosphatidic acid; lysoglycerophospholipids such as lysophosphatidylcholine (lysolecithin), lysophosphatidylethanolamine, lysophosphatidylserine, lysophosphatidylinositol, lysophosphatidylglycerol, and lysophosphatidic acid; sphingophospholipids such as sphingomyelin; and hydrogenated additives thereof. These phospholipids may be derived from animals or plants such as soybeans or egg yolks, or may be chemically or enzymatically synthesized.

Among the above-mentioned betaine compounds, preferred examples include lauryl dimethylaminoacetic acid betaine, lauramidopropyl betaine, lauryl hydroxysulfobetaine, stearyl betaine, lysophosphatidylcholine (lysolecithin), lysophosphatidylethanolamine, lysophosphatidylserine, lysophosphatidylinositol, lysophosphatidylglycerol, and lysophosphatidic acid; and more preferred examples include lysophosphatidylcholine (lysolecithin).

Examples of the ethylene glycol alkyl ether include polyoxyethylene alkyl ethers, polyoxyethylene lauryl ethers, polyoxyethylene palmitoyl ethers, and polyoxyethylene stearyl ethers. Commercially available ethylene glycol alkyl ethers may also be used, and examples of such commercial products include those from the EMULGEN (registered trademark) series and the EMANON (registered trademark) series available from Kao Corporation; for example, EMULGEN 102KG, EMULGEN 103, EMULGEN 104P, EMULGEN 105, EMULGEN 106, EMULGEN 108, EMULGEN 109P, EMULGEN 120, EMULGEN 123P, EMULGEN 130K, EMULGEN 147, EMULGEN 150, EMULGEN 210P, EMULGEN 220, EMULGEN 306P, EMULGEN 320P, EMULGEN 350, EMULGEN 404, EMULGEN 408, EMULGEN 409PV, EMULGEN 420, EMULGEN 430, EMULGEN 705, EMULGEN 707, EMULGEN 709, EMULGEN 1108, EMULGEN 1118S-70, EMULGEN 1135S-70, EMULGEN 1150S-60, EMULGEN 4085, EMULGEN 2020G-HA, EMULGEN 2025G, EMANON 1112, EMANON 3199V, EMANON 3299V, EMANON 3299RV, and EMANON 4110. More preferred examples include EMULGEN 103, EMULGEN 104P, EMULGEN 105, EMULGEN 106, EMULGEN 108, EMULGEN 109P, EMULGEN 210P, EMULGEN 306P, EMULGEN 320P, EMULGEN 404, EMULGEN 408, EMULGEN 409PV, EMULGEN 420, EMULGEN 705, EMULGEN 707, EMULGEN 709, EMULGEN 1108, EMULGEN 2020G-HA, EMANON 1112, and EMANON 4110 from Kao Corporation. Still more preferred examples include EMULGEN 104P, EMULGEN 105, EMULGEN 106, EMULGEN 108, EMULGEN 210P, EMULGEN 306P, EMULGEN 408, EMULGEN 409PV, EMULGEN 705, EMULGEN 707, EMULGEN 709, EMULGEN 1108, EMULGEN 2020G-HA, EMANON 1112, and EMANON 4110 from Kao Corporation. Besides the above, a suitable commercial product may be selected from the NIKKOL (registered trademark) series available from Nikko Chemicals Co., Ltd. Examples of suitable products from the NIKKOL series include NIKKOL BT-5, NIKKOL BT-7, NIKKOL BT-9, and NIKKOL BT-12.

Examples of the polyglycerin fatty acid ester include glyceryl fatty acid partial esters such as glyceryl stearate, glyceryl isostearate, glyceryl palmitate, glyceryl myristate, glyceryl oleate, glyceryl cocoate, glycerin mono-cottonseed oil fatty acid esters, glycerin monoerucate, glycerin sesquioleate, glycerin α,α'-oleate pyroglutamate, and glycerin monostearate malate; polyglyceryl-2 stearate, polyglyceryl-3 stearate, polyglyceryl-4 stearate, polyglyceryl-5 stearate, polyglyceryl-6 stearate, polyglyceryl-8 stearate, polyglyceryl-10 stearate, polyglyceryl-6 distearate, polyglyceryl-10 distearate, polyglyceryl-2 tristearate, polyglyceryl-10 decastearate, polyglyceryl-2 isostearate, polyglyceryl-3 isostearate, polyglyceryl-4 isostearate, polyglyceryl-5 isostearate, polyglyceryl-6 isostearate, polyglyceryl-8 isostearate, polyglyceryl-10 isostearate, polyglyceryl-2 diisostearate (diglyceryl diisostearate), polyglyceryl-3 diisostearate, polyglyceryl-10 diisostearate (decaglyceryl diisostearate), polyglyceryl-2 triisostearate, polyglyceryl-2 tetraisostearate, polyglyceryl-10 decaisostearate, polyglyceryl-2 oleate, polyglyceryl-3 oleate, polyglyceryl-4 oleate, polyglyceryl-5 oleate, polyglyceryl-6 oleate, polyglyceryl-8 oleate, polyglyceryl-10 oleate, polyglyceryl-6 dioleate, polyglyceryl-2 trioleate, and polyglyceryl-10 decaoleate.

Examples of the polyoxyethylene polyoxypropylene alkyl ether include EMULGEN (registered trademark) LS-106, EMULGEN LS-110, EMULGEN LS-114, and EMULGEN MS-110 from Kao Corporation; and NIKKOL (registered trademark) PBC-31, NIKKOL PBC-33, NIKKOL PBC-34, NIKKOL PBC-41, NIKKOL PBC-44, NIKKOL PBN-4612, NIKKOL PBN-4620, and NIKKOL PBN-4630 from Nikko Chemicals Co., Ltd. Preferred examples include EMULGEN LS-106, EMULGEN LS-110, EMULGEN LS-114, and EMULGEN MS-110. More preferred examples include EMULGEN LS-106, EMULGEN LS-110, and EMULGEN MS-110.

As the surfactant to be used in the present invention, a surfactant having an HLB (Hydrophile-Lipophile Balance) value of 8 to 20 can be suitably used. A surfactant having an HLB value of 8 to 14 is more preferred.

Examples of such surfactants include sorbitan isostearate, steareth-8, beheneth-10, laureth-5, ceteth-7, oleth-8, PEG-8 glyceryl isostearate, choleth-10, PEG-10BG isostearate, PEG-30 glyceryl triisostearate, PEG-30 glyceryl triisostearate, PEG-30 glyceryl trioleate, PEG-30 trimethylolpropane triisostearate, PEG-30 hydrogenated castor oil laurate, PEG-30 hydrogenated castor oil PCA isostearate, octyldodeceth-10, PEG-12 dilaurate, sorbeth-40 tetraoleate, polyglyceryl-10 diisostearate (decaglyceryl diisostearate), PEG-20 glyceryl diisostearate, PEG-8 isostearate, PEG-10 glyceryl isostearate, PEG-60 hydrogenated castor oil triisostearate, PPG-2-deceth-7, oleth-10, hydrogenated dimer dilinoleth-20, sorbitan cocoate, isosteareth-10, steareth-11, PEG-30 trimethylolpropane trimyristate, PEG-40 hydrogenated castor oil isostearate, PEG-40 hydrogenated castor oil isostearate, PEG-40 hydrogenated castor oil PCA isostearate, laureth-7, isoceteth-10, ceteth-10, PEG-10 isostearate, PEG-10 stearate, PEG-10 oleate, PEG-10 glyceryl stearate, oleth-12, decyltetradeceth-15, choleth-15, PEG-16 dilaurate, PEG-30 hydrogenated castor oil, PEG-40 glyceryl triisostearate, PEG-40 glyceryl trioleate, PEG-40 trimethylolpropane triisostearate, PEG-40 hydrogenated castor oil laurate, and PEG-12 laurate.

In the present invention, the amount of the surfactant to be contained is, for example, 0.5 to 20% by mass, preferably 0.5 to 10% by mass, and more preferably 0.5 to 5% by mass, based on the total mass of the obtained solid base material for external use on skin.

In the present invention, the amount of the surfactant to be contained is, for example, 1 to 20% by mass, and preferably 2 to 20% by mass, based on the total mass of the obtained aqueous composition.

The surfactant to be used in the present invention is at least one of the above-mentioned group of surfactants, and these surfactants may be used alone or in combination of two or more.

### [At Least One Saturated or Unsaturated Monohydric Alcohol Having a Carbon Atom Number of 8 to 30]

When at least one saturated or unsaturated monohydric alcohol having a carbon atom number of 8 to 30 is added to the solid base material for external use on skin or the aqueous composition, the thermal stability of the base material is significantly improved. The at least one saturated or unsaturated monohydric alcohol having a carbon atom number of 8 to 30 is also simply referred to herein as an "alkyl alcohol".

Many raw materials of these alkyl alcohols, including derivatives thereof, are commercially available or synthesized. In the composition, however, at least one saturated or unsaturated monohydric alcohol having a C₈₋₃₀ alkyl group may be preferably used, a saturated monohydric alcohol having a C₁₂₋₂₂ alkyl group may be more preferably used, and in particular, lauryl alcohol (C12), cetanol (C16), stearyl alcohol (C18), and behenyl alcohol (C22), which are highly versatile, are preferred. If the carbon atom number of the alkyl alcohol is excessively small, sufficient thermal stability cannot be achieved; conversely, if the carbon atom number of the alkyl alcohol is excessively great, the alkyl alcohol may not dissolve in the aqueous solution, and thus, a homogeneous composition may not be achieved.

In the present invention, the amount of the alkyl alcohol to be contained is, for example, 0.1 to 10% by mass, preferably 0.25 to 5% by mass, and more preferably 0.5 to 3% by mass, based on the total mass of the obtained solid base material for external use on skin.

In the present invention, the amount of the alkyl alcohol to be contained is, for example, 1 to 10% by mass, preferably 1 to 5% by mass, and more preferably 1 to 3% by mass, based on the total mass of the obtained aqueous composition.

The alkyl alcohol to be used in the present invention is at least one of the above-mentioned group of alkyl alcohols, and these alkyl alcohols may be used alone or in combination of two or more.

### [1,2-Alkanediolor 1,3-Alkanediol]

The solid base material for external use on skin or the aqueous composition may comprise a 1,2-alkanediol or 1,3-alkanediol. These alkanediols have the function of enhancing the solubility of the lipid peptide compound.

Specific examples of the 1,2-alkanediol include 1,2-pentanediol, 1,2-hexanediol, 1,2-octanediol, and 1,2-decanediol. Preferred examples include 1,2-pentanediol, 1,2-hexanediol, and 1,2-octanediol. More preferred is 1,2-pentanediol or 1,2-hexanediol. The 1,2-alkanediol to be used in the present invention is at least one of the above-mentioned group of 1,2-alkanediols.

Specific examples of the 1,3-alkanediol include 1,3-propanediol, 2-methyl-1,3-propanediol, 1,3-butanediol, 3-methyl-1,3-butanediol, 1,3-pentanediol, 1,3-hexanediol, 2-ethyl-1,3-hexanediol, 2-ethyl-1,3-octanediol, and 1,3-decanediol. Preferred examples include 1,3-pentanediol, 1,3-hexanediol, 2-ethyl-1,3-hexanediol, and 2-ethyl-1,3-octanediol. More preferred is 2-ethyl-1,3-hexanediol or 2-ethyl-1,3-octanediol. The 1,3-alkanediol to be used in the present invention is at least one of the above-mentioned group of 1,3-alkanediols.

These 1,2-alkanediols or 1,3-alkanediols may be used alone or in combination of two or more.

In the present invention, the amount of the 1,2-alkanediol or 1,3-alkanediol to be contained is, for example, 0.5 to 20% by mass, preferably 1 to 10% by mass, and more preferably 1 to 5% by mass, based on the total mass of the obtained solid base material for external use on skin.

In the present invention, the amount of the 1,2-alkanediol or 1,3-alkanediol to be contained is, for example, 2 to 20% by mass, and preferably 2 to 10% by mass, based on the total mass of the obtained aqueous composition.

### [Fatty Acid]

The solid base material for external use on skin or the aqueous composition may further comprise a fatty acid. The fatty acid to be used in the present invention is preferably at least one selected from the group consisting of saturated and unsaturated fatty acids each having a carbon atom number of 10 to 20, as well as salts of these fatty acids. Examples of fatty acids include capric acid, undecanoic acid, lauric acid, tridecanoic acid, myristic acid, pentadecanoic acid, palmitic acid, margaric acid, and stearic acid. More preferred examples include capric acid, lauric acid, myristic acid, palmitic acid, and stearic acid; in particular, stearic acid is preferred.

In the present invention, the amount of the fatty acid to be contained is, for example, 0.1 to 2.0% by mass, and preferably 0.2 to 1.0% by mass, based on the total mass of the obtained solid base material for external use on skin.

In the present invention, the amount of the fatty acid to be contained is, for example, 0.5 to 5% by mass, and preferably 0.5 to 3% by mass, based on the total mass of the obtained aqueous composition.

The fatty acid to be used in the present invention is at least one of the above-mentioned group of fatty acids, and these fatty acids may be used alone or in combination of two or more.

### [Oleaginous Base]

The solid base material for external use on skin may further comprise an oleaginous base. Likewise, the aqueous composition may further comprise an oleaginous base. Preferred examples of the oleaginous base to be used in the present invention include higher (polyhydric) alcohols such as oleyl alcohol, jojoba alcohol, chimyl alcohol, selachyl alcohol, batyl alcohol, hexyldecanol, isostearyl alcohol, 2-octyldodecanol, and dimer diols; aralkyl alcohols such as benzyl alcohol and derivatives thereof; isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteiso-heneicosanoic acid, branched long-chain fatty acids, dimer acids, hydrogenated dimer acids, and the like; hydrocarbons such as liquid paraffin (mineral oil), heavy liquid isoparaffin, light liquid isoparaffin, α-olefin oligomers, polyisobutene, hydrogenated polyisobutene, polybutene, squalane, olive-derived squalane, squalene, vaseline, and solid paraffin; waxes such as candelilla wax, carnauba wax, rice wax, Japan wax, beeswax, montan wax, ozokerite, ceresin, paraffin wax, microcrystalline wax, petrolatum, Fischer-Tropsch Wax, polyethylene wax, and ethylene-propylene copolymers; vegetable oils and fats such as coconut oil, palm oil, palm kernel oil, safflower oil, olive oil, castor oil, avocado oil, sesame oil, tea oil, evening primrose oil, wheat germ oil, macadamia nut oil, hazelnut oil, kukui nut oil, rose hip oil, meadowfoam oil, persic oil, tea tree oil, peppermint oil, corn oil, rapeseed oil, sunflower oil, wheat germ oil, linseed oil, cottonseed oil, soybean oil, peanut oil, rice bran oil, cacao butter, shea butter, hydrogenated coconut oil, hydrogenated castor oil, jojoba oil, and hydrogenated jojoba oil; animal oils and fats such as beef tallow, milk fat, horse fat, egg-yolk oil, mink oil, and turtle oil; animal waxes such as spermaceti, lanolin, and orange roughy oil; lanolins such as liquid lanolin, reduced lanolin, adsorption-purified lanolin, acetylated lanolin, acetylated liquid lanolin, hydroxylated lanolin, polyoxyethylene lanolin, lanolin fatty acids, hard lanolin fatty acids, lanolin alcohol, acetylated lanolin alcohol, and acetylated (cetyl/lanolyl) ester; sterols such as cholesterol, dihydrocholesterol, lanosterol, dihydrolanosterol, phytosterol, and cholic acid; sapogenins; saponins; sterol esters such as cholesteryl acetate, cholesteryl nonanoate, cholesteryl stearate, cholesteryl isostearate, cholesteryl oleate, di(cholesteryl/behenyl/octyldodecyl)N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, acyl sarcosine alkyl esters such as isopropyl N-lauroylsarcosinate, cholesteryl 12-hydroxystearate, cholesteryl macadamiate, phytosteryl macadamiate, phytosteryl isostearate, soft lanolin fatty acid cholesteryl esters, hard lanolin fatty acid cholesteryl esters, branched long-chain fatty acid cholesteryl esters, and long-chain α-hydroxy fatty acid cholesteryl esters; lipid complexes such as phospholipid-cholesterol complexes and phospholipid-phytosterol complexes; monohydric alcohol esters of carboxylic acids such as octyldodecyl myristate, hexyldecyl myristate, octyldodecyl isostearate, cetyl palmitate, octyldodecyl palmitate, cetyl octanoate, hexyldecyl octanoate, isotridecyl isononanoate, isononyl isononanoate, octyl isononanoate, isotridecyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, octyldodecyl lanolate, hexyldecyl dimethyloctanoate, octyldodecyl erucate, hydrogenated castor oil isostearate, ethyl oleate, ethyl avocadate, isopropyl myristate, isopropyl palmitate, octyl palmitate, isopropyl isostearate, isopropyl lanolate, diethyl sebacate, diisopropyl sebacate, dioctyl sebacate, diisopropyl adipate, dibutyloctyl sebacate, diisobutyl adipate, dioctyl succinate, and triethyl citrate; oxyacid esters such as cetyl lactate, diisostearyl malate, and hydrogenated castor oil monoisostearate; polyhydric alcohol esters of fatty acids such as glyceryl trioctanoate (glyceryl tri-2-ethylhexanoate), glyceryl trioleate, glyceryl triisostearate, glyceryl diisostearate, caprylic/capric triglyceride, caprylic/capric/myristic/stearic triglyceryl, hydrogenated rosin triglyceride (hydrogenated ester gum), rosin triglyceride (ester gum), glyceryl behenate eicosanedioate, trimethylolpropane trioctanoate, trimethylolpropane triisostearate, neopentyl glycol dioctanoate, neopentyl glycol dicaprate, 2-butyl-2-ethyl-1,3-propanediol dioctanoate, propylene glycol dioleate, pentaerythrityl tetraoctanoate, hydrogenated rosin pentaerythrityl ester, ditrimethylolpropane triethylhexanoate, ditrimethylolpropane isostearate/sebacate, pentaerythrityl triethylhexanoate, dipentaerythrityl hydroxystearate/stearate/rhodinate, diglyceryl diisostearate, polyglyceryl tetraisostearate, polyglyceryl-10 nonaisostearate, polyglyceryl-8 deca(erucate/isostearate/ricinoleate), (hexyldecanoic acid/sebacic acid) diglyceryl oligoester, glycol distearate (ethylene glycol distearate), 3-methyl-1,5-pentanediol dineopentanoate, and 2,4-diethyl-1,5-pentanediol dineopentanoate; dimer acid derivatives or dimer diol derivatives such as diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, dimer dilinoleyl diisostearate, dimer dilinoleyl hydrogenated rosin condensates, hydrogenated castor oil dimer dilinoleate, and hydroxyalkyl dimer dilinoleyl ether; fatty acid alkanolamides such as coconut oil fatty acid monoethanolamides (cocamide MEA), coconut oil fatty acid diethanolamides (cocamide DEA), lauric acid monoethanolamide (lauramide MEA), lauric acid diethanolamide (lauramide DEA), lauric acid monoisopropanolamide (lauramide MIPA), palmitic acid monoethanolamide (palmitamide MEA), palmitic acid diethanolamide (palmitamide DEA), and coconut oil fatty acid methylethanolamides (cocamide methyl MEA); silicones such as dimethicone (dimethylpolysiloxane), highly polymerized dimethicone (highly polymerized dimethylpolysiloxane), cyclomethicone (cyclic dimethylsiloxane, decamethylcyclopentasiloxane (also simply referred to as cyclopentasiloxane)), phenyl trimethicone, diphenyl dimethicone, phenyl dimethicone, stearoxypropyldimethylamine, (aminoethylaminopropyl methicone/dimethicone) copolymers, dimethiconol, dimethiconol crosspolymers, silicone resin, silicone rubber, amino-modified silicones such as aminopropyl dimethicone and amodimethicone, cation-modified silicones, polyether-modified silicones such as dimethicone copolyols, polyglycerin-modified silicones, sugar-modified silicones, carboxylic acid-modified silicones, phosphoric acid-modified silicones, sulfuric acid-modified silicones, alkyl-modified silicones, fatty acid-modified silicones, alkyl ether-modified silicones, amino acid-modified silicones, peptide-modified silicones, fluorine-modified silicones, cation-modified and polyether-modified silicones, amino-modified and polyether-modified silicones, alkyl-modified and polyether-modified silicones, and polysiloxane-oxyalkylene copolymers; and fluorine oils such as perfluorodecane, perfluorooctane, and perfluoropolyether.

In the present invention, the amount of the oleaginous base to be contained is, for example, 1 to 50% by mass, preferably 5 to 50% by mass, and more preferably 10 to 50% by mass, based on the total mass of the obtained solid base material for external use on skin.

In the present invention, when the aqueous composition comprises an oleaginous base, the amount of the oleaginous base to be contained is, for example, 50 to 1% by mass, and preferably 30 to 1% by mass, based on the total mass of the aqueous composition.

The oleaginous base to be used in the present invention is at least one of the above-mentioned group of oleaginous bases, and these oleaginous bases may be used alone or in combination of two or more.

### [Organic Acid]

The solid base material for external use on skin may further comprise an organic acid. Likewise, the aqueous composition may further comprise an organic acid.

Examples of the organic acid include ascorbic acid, citric acid, lactic acid, glycolic acid, succinic acid, acetic acid, malic acid, tartaric acid, and fumaric acid. Among the above, preferred examples include ascorbic acid, citric acid, and lactic acid, and particularly preferred examples include ascorbic acid and citric acid.

In the present invention, the amount of the organic acid to be contained is, for example, 1 to 20% by mass, and preferably 1 to 10% by mass, based on the total mass of the obtained solid base material for external use on skin.

Furthermore, in the present invention, when the aqueous composition comprises an organic acid, the amount of the organic acid to be contained is, for example, 1 to 20% by mass, and preferably 1 to 10% by mass, based on the total mass of the aqueous composition.

### [Polyhydric Alcohol]

The solid base material for external use on skin or the aqueous composition may comprise a polyhydric alcohol. The polyhydric alcohol is a polyhydric alcohol different from the earlier-mentioned 1,2-alkanediol or 1,3-alkanediol, and specific examples of the polyhydric alcohol include glycerin, propylene glycol, and polyethylene glycol. The inclusion of the polyhydric alcohol can improve the temporal stability of the solid base material for external use on skin. Polyethylene glycol with an average molecular weight of 1,000 to 4,000, for example, can be suitably used as the polyethylene glycol.

In the present invention, the amount of the polyhydric alcohol to be contained is, for example, 1 to 80% by mass, and preferably 1 to 60% by mass, based on the total mass of the obtained solid base material for external use on skin.

Furthermore, in the present invention, when the aqueous composition comprises a polyhydric alcohol, the amount of the polyhydric alcohol to be contained is, for example, 1 to 40% by mass, and preferably 1 to 20% by mass, based on the total mass of the aqueous composition.

### [Other Additives]

The solid base material for external use on skin or the aqueous composition may contain additives generally usable as additives for cosmetics, quasi-drugs, and pharmaceuticals, as required. Examples of additive ingredients such as physiologically active substances and functional substances contained in external preparations for skin such as cosmetics, quasi-drugs, or pharmaceuticals include moisturizers, texture improvers, surfactants other than those mentioned above, polymers, thickeners/gelators, solvents, propellants, antioxidants, reducing agents, oxidizing agents, preservatives, antimicrobial agents, antiseptics, chelating agents, pH adjusters, acids, alkalis, powders, inorganic salts, ultraviolet absorbers, whitening agents, vitamins and derivatives thereof, hair growth-promoting agents, blood circulation promoters, stimulants, hormones, anti-wrinkle agents, anti-aging agents, firming agents, cooling agents, warming agents, wound-healing promoters, abirritants, analgesics, cell activators, plant/animal/microbial extracts, antipruritics, exfoliates/keratolytic agents, antiperspirants, refrigerants, astringents, enzymes, nucleic acids, perfumes, colors, coloring agents, dyes, pigments, antiphlogistics, anti-inflammatory agents, anti-asthmatic agents, anti-chronic obstructive pulmonary disease agents, anti-allergic agents, immunomodulators, anti-infective agents, and antifungal agents.

The amount of these other additives to be contained may vary depending on the types of the additives; however, it is, for example, about 0.1 to 20% by mass or about 0.5 to 10% by mass, based on the total mass of the obtained solid base material for external use on skin.

Preferred examples of moisturizers and texture improvers include polyols and polymers thereof such as glycerin, trimethylolpropane, pentaerythritol, hexylene glycol, diglycerin, polyglycerin, diethylene glycol, dipropylene glycol, polypropylene glycol, and ethylene glycol-propylene glycol copolymers; glycol alkyl ethers such as diethylene glycol monoethyl ether (ethoxydiglycol), ethylene glycol monoethyl ether, ethylene glycol monobutyl ether, and diethylene glycol dibutyl ether; water-soluble esters such as polyglyceryl-10 (eicosanedioate/tetradecanedioate) and polyglyceryl-10 tetradecanedioate; sugar alcohols such as sorbitol, xylitol, erythritol, mannitol, and maltitol; saccharides and derivatives thereof such as glucose, fructose, galactose, mannose, threose, xylose, arabinose, fucose, ribose, deoxyribose, maltose, trehalose, lactose, raffinose, gluconic acid, glucuronic acid, cyclodextrins (α-, β-, and γ-cyclodextrins, and modified cyclodextrins such as maltosyl cyclodextrin and hydroxyalkyl cyclodextrin), β-glucan, chitin, chitosan, heparin and derivatives thereof, pectin, arabinogalactan, dextrin, dextran, glycogen, ethyl glucoside, and glucosylethyl methacrylate polymer or copolymer; hyaluronic acid and sodium hyaluronate; sodium chondroitin sulfate; mucoitin sulfate, charonin sulfate, kerato sulfate, and dermatan sulfate; *Tremella fuciformis* extract and *Tremella fuciformis* polysaccharides; fucoidan; tuberose polysaccharides or natural polysaccharides; organic acids such as citric acid, tartaric acid, and lactic acid, as well as salts thereof; urea and derivatives thereof; 2-pyrrolidone-5-carboxylic acid and salts thereof such as sodium salt; amino acids such as betaine (trimethylglycine), proline, hydroxyproline, arginine, lysine, serine, glycine, alanine, phenylalanine, tyrosine, β-alanine, threonine, glutamic acid, glutamine, asparagine, aspartic acid, cystine, cysteine, methionine, leucine, isoleucine, valine, tryptophan, histidine, and taurine, as well as salts thereof; protein peptides and derivatives thereof such as collagen, fish collagen, atelocollagen, gelatin, elastin, peptides derived from degraded collagen, hydrolyzed collagen, hydroxypropylammonium chloride hydrolyzed collagen, peptides derived from degraded elastin, peptides derived from degraded keratin, hydrolyzed keratin, peptides derived from degraded conchiolin, hydrolyzed conchiolin, peptides derived from degraded silk protein, hydrolyzed silk, sodium lauroyl hydrolyzed silk, peptides derived from degraded soy protein, peptides derived from degraded wheat protein, hydrolyzed wheat protein, peptides derived from degraded casein, and acylated peptides; acylated peptides such as palmitoyl oligopeptide, palmitoyl pentapeptide, and palmitoyl tetrapeptide; silylated peptides; lactic acid bacteria culture, yeast extracts, eggshell membrane protein, bovine submaxillary mucin, hypotaurine, sesame lignan glycosides, glutathione, albumin, and whey; choline chloride and phosphorylcholine; animal and plant extract components such as placenta extract, elastin, collagen, aloe extract, *Hammamelis virginiana* water, *Luffa cylindrica* water, *Chamomilla recutita* extract, licorice extract, comfrey extract, silk extract, *Rosa roxburghii* extract, *Achillea millefolium* extract, *Eucalyptus globulus* extract, and melilot extract; and ceramides such as natural ceramides (types 1, 2, 3, 4, 5, and 6), hydroxyceramide, pseudoceramide, sphingoglycolipid, ceramide-containing extract, and glucosylceramide-containing extract.

Preferred examples of surfactants include anionic surfactants, nonionic surfactants, cationic surfactants, amphoteric surfactants, and polymer surfactants. Preferred examples of these surfactants are as follows: Preferred examples of anionic surfactants include fatty acid salts such as potassium laurate and potassium myristate; alkyl sulfates such as sodium lauryl sulfate, triethanolamine lauryl sulfate, and ammonium lauryl sulfate; polyoxyethylene alkyl sulfates such as sodium laureth sulfate and triethanolamine laureth sulfate; acyl N-methylamino acid salts such as sodium cocoyl methyl taurate, potassium cocoyl methyl taurate, sodium lauroyl methyl taurate, sodium myristoyl methyl taurate, sodium lauroyl methyl alaninate, sodium lauroyl sarcosinate, triethanolamine lauroyl sarcosinate, and sodium lauroyl glutamate methyl alaninate; acyl amino acid salts such as sodium cocoyl glutamate, triethanolamine cocoyl glutamate, sodium lauroyl glutamate, sodium myristoyl glutamate, sodium stearoyl glutamate, ditriethanolamine palmitoyl aspartate, and triethanolamine cocoyl alaninate; polyoxyethylene alkyl ether acetates such as sodium laureth acetate; succinates such as sodium lauroyl monoethanolamide succinate; fatty acid alkanolamide ether carboxylates; acyl lactates; polyoxyethylene fatty amine sulfates; fatty acid alkanolamide sulfates; fatty acid glyceride sulfates such as sodium hydrogenated coconut oil fatty acid glycerin sulfates; alkylbenzene polyoxyethylene sulfates; olefin sulfonates such as sodium α-olefin sulfonates; alkyl sulfosuccinates such as disodium lauryl sulfosuccinate and sodium dioctyl sulfosuccinate; alkyl ether sulfosuccinates such as disodium laureth sulfosuccinate, sodium monolauroyl monoethanolamide polyoxyethylene sulfosuccinate, and sodium lauryl polypropylene glycol sulfosuccinate; alkylbenzene sulfonates such as sodium tetradecylbenzene sulfonate and triethanolamine tetradecylbenzene sulfonate; alkyl naphthalene sulfonates; alkane sulfonates; α-sulfofatty acid methyl ester salts; acyl isethionates; alkyl glycidyl ether sulfonates; alkyl sulfoacetates; alkyl ether phosphates such as sodium laureth phosphate, sodium dilaureth phosphate, sodium trilaureth phosphate, and sodium monooreth phosphate; alkyl phosphates such as potassium lauryl phosphate; sodium caseinate; alkyl aryl ether phosphates; fatty acid amide ether phosphates; phospholipids such as phosphatidylglycerol, phosphatidylinositol, and phosphatidic acid; and silicone anionic surfactants such as carboxylic acid-modified silicones, phosphoric acid-modified silicones, and sulfuric acid-modified silicones. Preferred examples of nonionic surfactants include polyoxyethylene alkyl ethers with various numbers of polyoxyethylene units, such as laureths (polyoxyethylene lauryl ethers), ceteths (polyoxyethylene cetyl ethers), steareths (polyoxyethylene stearyl ethers), beheneths (polyoxyethylene behenyl ethers), isosteareths (polyoxyethylene isostearyl ethers), and octyldodeceths (polyoxyethylene octyldodecyl ethers); polyoxyethylene alkyl phenyl ethers; castor oil derivatives and hydrogenated castor oil derivatives such as polyoxyethylene hydrogenated castor oil, polyoxyethylene castor oil, polyoxyethylene hydrogenated castor oil monoisostearate, polyoxyethylene hydrogenated castor oil triisostearate, polyoxyethylene hydrogenated castor oil monopyroglutamate monoisostearate diester, and polyoxyethylene hydrogenated castor oil maleate; polyoxyethylene phytosterol; polyoxyethylene cholesterol; polyoxyethylene cholestanol; polyoxyethylene lanolin; polyoxyethylene reduced lanolin; polyoxyethylene-polyoxypropylene alkyl ethers such as polyoxyethylene-polyoxypropylene cetyl ether, polyoxyethylene-polyoxypropylene 2-decyltetradecyl ether, polyoxyethylene-polyoxypropylene monobutyl ether, polyoxyethylene-polyoxypropylene hydrogenated lanolin, and polyoxyethylene-polyoxypropylene glycerin ether; polyoxyethylene-polyoxypropylene glycol; (poly)glycerin polyoxypropylene glycols such as PPG-9 diglyceryl; glycerol fatty acid partial esters such as glyceryl stearate, glyceryl isostearate, glyceryl palmitate, glyceryl myristate, glyceryl oleate, glyceryl cocoate, glycerin mono-cottonseed oil fatty acid esters, glycerin monoerucate, glycerin sesquioleate, glycerin α,α'-oleate pyroglutamate, and glycerin monostearate malate; polyglycerin fatty acid esters such as polyglyceryl-2 stearate, polyglyceryl-3 stearate, polyglyceryl-4 stearate, polyglyceryl-5 stearate, polyglyceryl-6 stearate, polyglyceryl-8 stearate, polyglyceryl-10 stearate, polyglyceryl-6 distearate, polyglyceryl-10 distearate, polyglyceryl-2 tristearate, polyglyceryl-10 decastearate, polyglyceryl-2 isostearate, polyglyceryl-3 isostearate, polyglyceryl-4 isostearate, polyglyceryl-5 isostearate, polyglyceryl-6 isostearate, polyglyceryl-8 isostearate, polyglyceryl-10 isostearate, polyglyceryl-2 diisostearate (diglyceryl diisostearate), polyglyceryl-3 diisostearate, polyglyceryl-10 diisostearate, polyglyceryl-2 triisostearate, polyglyceryl-2 tetraisostearate, polyglyceryl-10 decaisostearate, polyglyceryl-2 oleate, polyglyceryl-3 oleate, polyglyceryl-4 oleate, polyglyceryl-5 oleate, polyglyceryl-6 oleate, polyglyceryl-8 oleate, polyglyceryl-10 oleate, polyglyceryl-6 dioleate, polyglyceryl-2 trioleate, and polyglyceryl-10 decaoleate; ethylene glycol mono-fatty acid esters such as ethylene glycol monostearate; propylene glycol mono-fatty acid esters such as propylene glycol monostearate; pentaerythritol fatty acid partial esters; sorbitol fatty acid partial esters; maltitol fatty acid partial esters; maltitol ether; sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; sugar derivative partial esters such as sucrose fatty acid esters, methyl glucoside fatty acid esters, and trehalose undecylenoate; alkyl glucosides such as caprylyl glucoside; alkyl polyglycosides; lanolin alcohol; reduced lanolin; polyoxyethylene fatty acid mono- and di-esters such as polyoxyethylene distearate, polyethylene glycol diisostearate, polyoxyethylene monooleate, and polyoxyethylene dioleate; polyoxyethylene-propylene glycol fatty acid esters; polyoxyethylene glycerin fatty acid esters, for example, polyoxyethylene monooleates such as polyoxyethylene glycerin monostearate, polyoxyethylene glycerin monoisostearate, and polyoxyethylene glycerin triisostearate; polyoxyethylene sorbitan fatty acid esters such as polyoxyethylene sorbitan monooleate, polyoxyethylene sorbitan monostearate, polyoxyethylene sorbitan monooleate, and polyoxyethylene sorbitan tetraoleate; polyoxyethylene sorbitol fatty acid esters such as polyoxyethylene sorbitol monolaurate, polyoxyethylene sorbitol monooleate, polyoxyethylene sorbitol pentaoleate, and polyoxyethylene sorbitol monostearate; polyoxyethylene methyl glucoside fatty acid esters; polyoxyethylene alkyl ether fatty acid esters; polyoxyethylene-modified animal and vegetable fats and oils such as polyoxyethylene sorbitol beeswax; alkyl glyceryl ethers such as isostearyl glyceryl ether, chimyl alcohol, selachyl alcohol, and batyl alcohol; polyhydric alcohol alkyl ethers; polyoxyethylene alkylamines; tetrapolyoxyethylene/tetrapolyoxypropylene-ethylenediamine condensates; natural surfactants such as saponin and sophorolipid; polyoxyethylene fatty acid amides; fatty acid alkanolamides such as coconut oil fatty acid monoethanolamides (cocamide MEA), coconut oil fatty acid diethanolamides (cocamide DEA), lauric acid monoethanolamide (lauramide MEA), lauric acid diethanolamide (lauramide DEA), lauric acid monoisopropanolamide (lauramide MIPA), palmitic acid monoethanolamide (palmitamide MEA), palmitic acid diethanolamide (palmitamide DEA), and coconut oil fatty acid methylethanolamides (cocamide methyl MEA); alkyl dimethylamine oxides such as lauramine oxide, cocamine oxide, stearamine oxide, and behenamine oxide; alkyl ethoxydimethylamine oxides; polyoxyethylene alkyl mercaptans; and silicone nonionic surfactants, for example, polyether-modified silicones such as dimethicone copolyols, polysiloxane-oxyalkylene copolymers, polyglycerin-modified silicones, and sugar-modified silicones. Preferred examples of cationic surfactants include alkyl trimethylammonium chlorides such as behentrimonium chloride, steartrimonium chloride, cetrimonium chloride, and lauryltrimonium chloride; alkyl trimethylammonium bromides such as steartrimonium bromide; dialkyl dimethylammonium chlorides such as distearyldimonium chloride and dicocodimonium chloride; fatty acid amide amines such as stearamidopropyl dimethylamine and stearamidoethyl diethylamine, as well as salts thereof; alkyl ether amines such as stearoxypropyldimethylamine and salts or quaternary salts thereof; fatty acid amide quaternary ammonium salts such as branched long-chain fatty acid (12 to 31) aminopropylethyldimethylammonium ethyl sulfates and lanolin fatty acid aminopropylethyldimethylammonium ethyl sulfates; polyoxyethylene alkylamines and salts or quaternary salts thereof; alkylamine salts; fatty acid amide guanidium salts; alkyl ether amine ammonium salts; alkyl trialkylene glycol ammonium salts; benzalkonium salts; benzethonium salts; pyridinium salts such as cetylpyridinium chloride; imidazolinium salts; alkyl isoquinolinium salts; dialkyl morpholinium salts; polyamine fatty acid derivatives; and silicone cationic surfactants such as amino-modified silicones such as aminopropyl dimethicone and amodimethicone, cation-modified silicones, cation-modified and polyether-modified silicones, and amino-modified and polyether-modified silicones. Preferred examples of amphoteric surfactants include N-alkyl-N,N-dimethylamino acid betaines such as lauryl betaine (lauryl dimethylaminoacetic acid betaine); fatty acid amido alkyl-N,N-dimethylamino acid betaines such as cocamidopropyl betaine and lauramidopropyl betaine; imidazoline betaines such as sodium cocoamphoacetate and sodium lauroamphoacetate; alkyl sulfobetaines such as alkyl dimethyltaurine; betaine sulfates such as alkyl dimethylaminoethanol sulfate; betaine phosphates such as alkyl dimethylaminoethanol phosphates; phospholipids such as phosphatidylcholine, phosphatidylethanolamine, phosphatidylserine, sphingophospholipids such as sphingomyelin, lysolecithin, hydrogenated soybean phospholipid, partially hydrogenated soybean phospholipid, hydrogenated egg yolk phospholipid, partially hydrogenated egg yolk phospholipid, and hydroxylated lecithin; and silicone amphoteric surfactants. Preferred examples of polymer surfactants include polyvinyl alcohol, sodium alginate, starch derivatives, tragacanth gum, and acrylic acid-alkyl methacrylate copolymers; and various silicone surfactants.

Preferred examples of polymers, thickeners, and gelators include guar gum, locust bean gum, quince seed, carrageenan, galactan, gum arabic, tara gum, tamarind, furcellaran, karaya gum, *Abelmoschus manihot,* cara gum, tragacanth gum, pectin, pectic acid and salts thereof such as sodium salt, alginic acid and salts thereof such as sodium salt, and mannan; starches such as rice starch, corn starch, potato starch, and wheat starch; xanthan gum, dextran, succinoglucan, curdlan, hyaluronic acid and salts thereof, xanthan gum, pullulan, gellan gum, chitin, chitosan, agar, brown algae extract, chondroitin sulfate, casein, collagen, gelatin, and albumin; celluloses and derivatives thereof such as methyl cellulose, ethyl cellulose, hydroxyethyl cellulose, hydroxypropyl cellulose, hydroxypropyl methylcellulose, carboxymethyl cellulose and salts thereof such as sodium salt, methylhydroxypropyl cellulose, sodium cellulose sulfate, dialkyldimethylammonium cellulose sulfate, crystalline cellulose, and cellulose powder; starch derivatives such as soluble starch, starch polymers such as carboxymethyl starch, methylhydroxypropyl starch, and methyl starch, starch hydroxypropyltrimonium chloride, and aluminum corn starch octenylsuccinate; alginic acid derivatives such as sodium alginate and propylene glycol alginate; polyvinylpyrrolidone (PVP), polyvinyl alcohol (PVA), vinylpyrrolidone-vinyl alcohol copolymers, and polyvinyl methyl ether; polyethylene glycol, polypropylene glycol, and polyoxyethylene-polyoxypropylene copolymers; amphoteric methacrylic acid ester copolymers such as (methacryloyloxyethylcarboxybetaine/alkyl methacrylate) copolymers and (acrylate/stearyl acrylate/ethylamine oxide methacrylate) copolymers; (dimethicone/vinyl dimethicone) crosspolymers, (alkyl acrylate/diacetone acrylamide) copolymers, and (alkyl acrylate/diacetone acrylamide) copolymers AMP; partially saponified polyvinyl acetate and maleic acid copolymers; vinylpyrrolidone-dialkylaminoalkyl methacrylate copolymers; acrylic resin alkanolamine; polyester and water-dispersible polyester; polyacrylamide; copolymers ofpolyacrylic esters such as polyethyl acrylate, carboxy vinyl polymers, polyacrylic acid and salts thereof such as sodium salt, and acrylic acid-methacrylic acid ester copolymers; acrylic acid-alkyl methacrylate copolymers; cationized celluloses such as polyquaternium-10, diallyldimethylammonium chloride-acrylamide copolymers such as polyquaternium-7, acrylic acid-diallyldimethylammonium chloride copolymers such as polyquaternium-22, acrylic acid-diallyldimethylammonium chloride-acrylamide copolymers such as polyquaternium-39, acrylic acid-cationized methacrylic acid ester copolymers, acrylic acid-cationized methacrylic acid amide copolymers, acrylic acid-methyl acrylate-methacrylamidopropyltrimethylammonium chloride copolymers such as polyquaternium-47, and methacryloyl chloride choline ester polymers; cationized polysaccharides such as cationized oligosaccharides, cationized dextran, and guar hydroxypropyltrimonium chloride; polyethyleneimine; cationic polymers; copolymers of 2-methacryloyloxyethyl phosphorylcholine and butyl methacrylate, such as polyquaternium-51; polymer emulsions such as acrylic resin emulsions, poly(ethyl acrylate) emulsions, polyacrylalkyl ester emulsions, polyvinyl acetate resin emulsions, natural rubber latex, and synthetic latex; nitrocellulose; polyurethanes and various copolymers thereof; various silicones; various silicone copolymers such as acrylic-silicone graft copolymers; various fluorine polymers; 12-hydroxystearic acid and salts thereof; dextrin fatty acid esters such as dextrin palmitate and dextrin myristate; and silicic anhydride, fumed silica (silicic anhydride ultrafine particles), magnesium aluminum silicate, magnesium sodium silicate, metallic soaps, metal dialkyl phosphates, bentonite, hectorite, organo-modified clay minerals, sucrose fatty acid esters, and fructooligosaccharide fatty acid esters. Among the above-mentioned examples, cellulose and derivatives thereof, alginic acid and salts thereof, polyvinyl alcohol, hyaluronic acid and salts thereof, and collagen are preferred.

Preferred examples of solvents and propellants include lower alcohols such as ethanol, 2-propanol (isopropyl alcohol), butanol, and isobutyl alcohol; glycols such as propylene glycol, diethylene glycol, dipropylene glycol, and isopentyldiol; glycol ethers such as diethylene glycol monoethyl ether (ethoxy diglycol), ethylene glycol monoethyl ether, ethylene glycol monobutyl ether, triethylene glycol monoethyl ether, diethylene glycol diethyl ether, diethylene glycol dibutyl ether, propylene glycol monoethyl ether, and dipropylene glycol monoethyl ether; glycol ether esters such as ethylene glycol monoethyl ether acetate, diethylene glycol monoethyl ether acetate, and propylene glycol monoethyl ether acetate; glycol esters such as diethoxyethyl succinate and ethylene glycol disuccinate; benzyl alcohol, benzyloxyethanol, propylene carbonate, dialkyl carbonate, acetone, ethyl acetate, and N-methylpyrrolidone; toluene; fluorocarbons and next-generation fluorocarbons; and propellants such as LPG, dimethyl ether, and carbon dioxide gas.

Preferred examples of antioxidants include tocopherol (vitamin E) and tocopherol derivatives such as tocopherol acetate; BHT and BHA; gallic acid derivatives such as propyl gallate; vitamin C (ascorbic acid) and/or derivatives thereof; erythorbic acid and derivatives thereof; sulfites such as sodium sulfite; hydrogen sulfites such as sodium hydrogen sulfite; thiosulfates such as sodium thiosulfate; metabisulfites; thiotaurine and hypotaurine; thioglycerol, thiourea, thioglycolic acid, and cysteine hydrochloride.

Preferred examples of reducing agents include thioglycolic acid, cysteine, and cysteamine.

Preferred examples of oxidizing agents include hydrogen peroxide solution, ammonium persulfate, sodium bromate, and percarbonic acid.

Preferred examples of preservatives, antimicrobial agents, and antiseptics include hydroxybenzoic acids such as methylparaben, ethylparaben, propylparaben, and butylparaben, as well as salts or esters thereof; salicylic acid; sodium benzoate; phenoxyethanol; isothiazolinone derivatives such as methylchloroisothiazolinone and methylisothiazolinone; imidazoliniumurea; dehydroacetic acid and salts thereof; phenols; halogenated bisphenols such as triclosan, acid amides thereof, and quarternary ammonium salts thereof; trichlorocarbanide, zinc pyrithione, benzalkonium chloride, benzethonium chloride, sorbic acid, chlorhexidine, chlorhexidine gluconate, halocarban, hexachlorophene, and hinokitiol; other phenols such as phenol, isopropylphenol, cresol, thymol, parachlorohenol, phenylphenol, and sodium phenylphenate; phenyl ethyl alcohol, photosensitizers, antibacterial zeolite, and silver ions.

Preferred examples of chelating agents include edetates (ethylenediamine tetraacetates) such as EDTA, EDTA-2Na, EDTA-3Na, and EDTA-4Na; hydroxyethylethylenediamine triacetates such as HEDTA-3Na; pentetates (diethylenetriamine pentaacetate); phytic acid; phosphonic acids such as etidronic acid and salts thereof such as sodium salt; polyamino acids such as polyaspartic acid and polyglutamic acid; sodium polyphosphate, sodium metaphosphate, and phosphoric acid; sodium citrate, citric acid, alanine, dihydroxyethylglycine, gluconic acid, ascorbic acid, succinic acid, and tartaric acid.

Preferred examples of pH adjusters, acids, and alkalis include citric acid, sodium citrate, lactic acid, sodium lactate, potassium lactate, glycolic acid, succinic acid, acetic acid, sodium acetate, malic acid, tartaric acid, fumaric acid, phosphoric acid, hydrochloric acid, sulfuric acid, monoethanolamine, diethanolamine, triethanolamine, isopropanolamine, triisopropanolamine, 2-amino-2-methyl-1,3-propanediol, 2-amino-2-hydroxymethyl-1,3-propanediol, arginine, sodium hydroxide, potassium hydroxide, aqueous ammonia, guanidine carbonate, and ammonium carbonate.

Preferred examples of powders include inorganic powders having various sizes and shapes, such as mica, talc, kaolin, sericite, montmorillonite, kaolinite, mica, muscovite, phlogopite, synthetic mica, lepidolite, biotite, vermiculite, magnesium carbonate, calcium carbonate, aluminum silicate, barium silicate, calcium silicate, magnesium silicate, strontium silicate, metal tungstates, magnesium, zeolite, barium sulfate, calcined calcium sulfate, calcium phosphate, fluorapatite, hydroxyapatite, ceramic powder, bentonite, smectite, clay, mud, metallic soaps (for example, zinc myristate, calcium palmitate, and aluminum stearate), calcium carbonate, red iron oxide, yellow iron oxide, black iron oxide, ultramarine, prussian blue, carbon black, titanium oxide, titanium oxide fine particles and titanium oxide ultrafine particles, zinc oxide, zinc oxide fine particles and zinc oxide ultrafine particles, alumina, silica, fumed silica (silicic anhydride ultrafine particles), titanated mica, fish scale foil, boron nitride, photochromic pigments, synthetic fluorophlogopites, particulate composite powders, gold, and aluminum, as well as inorganic powders such as hydrophobic or hydrophilic powders obtained by treating the above-mentioned powders with various surface-treating agents such as silicones, for example, hydrogen silicone and cyclic hydrogen silicone, other silanes, or titanium coupling agents; and organic powders, surface-treated powders thereof, and organic-inorganic composite powders having various sizes and shapes, such as starches, celluloses, nylon powders, polyethylene powders, polymethyl methacrylate powders, polystyrene powders, styrene-acrylic acid copolymer resin powders, polyester powders, benzoguanamine resin powders, polyethylene terephthalate-polymethyl methacrylate laminated powders, polyethylene terephthalate-aluminum-epoxy laminated powders, urethane powders, silicone powders, and Teflon (registered trademark) powder.

Preferred examples of inorganic salts include sodium chloride-containing salts such as common salt, regular salt, rock salt, sea salt, and natural salt; potassium chloride, aluminum chloride, calcium chloride, magnesium chloride, bittern, zinc chloride, and ammonium chloride; sodium sulfate, aluminum sulfate, aluminum potassium sulfate (alum), aluminum ammonium sulfate, barium sulfate, calcium sulfate, potassium sulfate, magnesium sulfate, zinc sulfate, iron sulfate, and copper sulfate; and sodium phosphates such as mono-, di-, and tri-sodium phosphates, potassium phosphates, calcium phosphates, and magnesium phosphates.

Preferred examples of ultraviolet absorbers include benzoate ultraviolet absorbers such as p-aminobenzoic acid, p-aminobenzoic acid monoglycerin ester, N,N-dipropoxy-p-aminobenzoic acid ethyl ester, N,N-diethoxy-p-aminobenzoic acid ethyl ester, N,N-dimethyl-p-aminobenzoic acid ethyl ester, N,N-dimethyl-p-aminobenzoic acid butyl ester, and N,N-dimethyl-p-aminobenzoic acid methyl ester; anthranilate ultraviolet absorbers such as homomenthyl-N-acetylanthranilate; salicylate ultraviolet absorbers such as salicylic acid and sodium salt thereof, amyl salicylate, methyl salicylate, homomenthyl salicylate, octyl salicylate, phenyl salicylate, benzyl salicylate, and p-isopropanolphenyl salicylate; cinnamate ultraviolet absorbers such as octyl cinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, 2-ethylhexyl-p-methoxycinnamate (octyl p-methoxycinnamate), 2-ethoxyethyl-p-methoxycinnamate (cinoxate), cyclohexyl-p-methoxycinnamate, ethyl-α-cyano-β-phenylcinnamate, 2-ethylhexyl-α-cyano-β-phenylcinnamate (octocrylene), glyceryl mono-2-ethylhexanoyl-di-p-methoxycinnamate, ferulic acid, and derivatives thereof; benzophenone ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone (oxybenzone-3), 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone; 3-(4'-methylbenzylidene)-d,l-camphor and 3-benzylidene-d,1-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenyl)benzotriazole; dibenzalazine; dianisoylmethane; 5-(3,3-dimethyl-2-norbornylidene)-3-pentan-2-one; dibenzoylmethane derivatives such as 4-t-butyl methoxydibenzoylmethane; octyl triazone; urocanic acid and urocanic acid derivatives such as ethyl urocanate; and 2-(2'-hydroxy-5'-methylphenyl)benzotriazole, 1-(3,4-dimethoxyphenyl)-4,4-dimethyl-1,3-pentanedione, hydantoin derivatives such as 2-ethylhexyl dimethoxybenzylidene dioxoimidazolidine propionate, phenylbenzimidazole sulfonic acid, terephthalylidene dicamphor sulfonic acid, drometrizole trisiloxane, methyl anthranilate, rutin and derivatives thereof, and oryzanol and derivatives thereof.

Preferred examples of whitening agents include hydroquinone glycosides such as arbutin and α-arbutin, as well as esters thereof; ascorbic acid and ascorbic acid derivatives, for example, ascorbyl phosphates such as sodium ascorbyl phosphate and magnesium ascorbyl phosphate, ascorbyl fatty acid esters such as ascorbyl tetraisopalmitate, ascorbic acid alkyl ethers such as ascorbic acid ethyl ether, ascorbic acid glucosides such as ascorbic acid 2-glucoside and fatty acid esters thereof, ascorbyl sulfate, and tocopheryl ascorbyl phosphate; kojic acid, ellagic acid, tranexamic acid and derivatives thereof, ferulic acid and derivatives thereof, placenta extract, glutathione, oryzanol, butylresorecinol, and plant extracts such as oil-soluble *Chamomilla recutita* flower extract, oil-soluble licorice extract, *Tamarix chinensis* extract, and *Saxifraga stolonifera* extract.

Preferred examples of vitamins and derivatives thereof include forms of vitamin A such as retinol, retinol acetate, and retinol palmitate; the vitamin B group such as thiamine hydrochloride, thiamine sulfate, riboflavin, riboflavin acetate, pyridoxine hydrochloride, pyridoxine dioctanoate, pyridoxine dipalmitate, flavin adenine dinucleotide, cyanocobalamin, folic acids, nicotinic acids such as nicotinamide and benzyl nicotinate, and cholines; forms of vitamin C such as ascorbic acid and salts thereof such as sodium salt; vitamin D; forms of vitamin E such as α-, β-, γ-, and δ-tocopherols; other vitamins such as pantothenic acid and biotin; ascorbic acid derivatives, for example, ascorbyl phosphates such as sodium ascorbyl phosphate and magnesium ascorbyl phosphate, ascorbyl fatty acid esters such as ascorbyl tetraisopalmitate, ascorbyl stearate, ascorbyl palmitate, and ascorbyl dipalmitate, ascorbic acid alkyl ethers such as ascorbic acid ethyl ether, ascorbic acid glucosides such as ascorbic acid 2-glucoside and fatty acid esters thereof, and tocopheryl ascorbyl phosphate; vitamin derivatives, for example, tocopherol derivatives such as tocopherol nicotinate, tocopherol acetate, tocopherol linoleate, tocopherol ferulate, and tocopherol phosphate, tocotrienol, and other various vitamin derivatives.

Preferred examples of hair growth-promoting agents, blood circulation promoters, and stimulants include plant extracts and tinctures such as swertia herb extract, capsicum tincture, ginger tincture, ginger extract, and cantharis tincture; capsaicin, nonylic acid vanillylamide, zingerone, ichthammol, tannic acid, borneol, cyclandelate, cinnarizine, tolazoline, acetylcholine, verapamil, cepharanthine, γ-oryzanol, vitamin E and derivatives thereof such as tocopherol nicotinate and tocopherol acetate, γ-oryzanol, nicotinic acid and derivatives thereof such as nicotinamide, benzyl nicotinate, inositol hexanicotinate, and nicotinic alcohol, allantoin, Photosensitizer 301, Photosensitizer 401, carpronium chloride, pentadecanoic acid monoglyceride, flavanonol derivatives, stigmasterol or stigmastanol and glycosides thereof, and minoxidil.

Preferred examples of hormones include estradiol, estrone, ethinylestradiol, cortisone, hydrocortisone, and prednisone. Preferred examples of other medicinal agents such as anti-wrinkle agents, anti-aging agents, firming agents, cooling agents, warming agents, wound-healing promoters, abirritants, analgesics, and cell activators include retinols, retinoic acids, and tocopheryl retinoate; lactic acid, glycolic acid, gluconic acid, fruit acid, salicylic acid, and derivatives thereof such as glycosides and esters, and α- or β-hydroxy acids and derivatives thereof such as hydroxycapric acid, long-chain α-hydroxy fatty acids, and long-chain α-hydroxy fatty acid cholesteryl esters; γ-aminobutyric acid and γ-amino-β-hydroxybutyric acid; carnitine; carnocin; creatine; ceramides and sphingosines; caffeine, xanthine, and the like, as well as derivatives thereof; anti-oxidizing agents and active oxygen scavengers such as coenzyme Q10, carotene, lycopene, astaxanthin, lutein, α-lipoic acid, colloidal platinum nanoparticles, and fullerenes; catechins; flavones such as quercetin; isoflavones; gallic acid and sugar ester derivatives thereof; polyphenols such as tannin, sesamin, proanthocyanidin, chlorogenic acid, and apple polyphenols; rutin and derivatives thereof such as glycosides; hesperidin and derivatives thereof such as glycosides; lignan glycosides; licorice extract-related substances such as glabridin, glabrene, liquiritin, and isoliquiritin; lactoferrin; shogaol and gingerol; perfume substances such as menthol and cedrol as well as derivatives thereof; capsaicin, vanillin, and the like, as well as derivatives thereof; insect repellents such as diethyltoluamide; and complexes of physiologically active substances and cyclodextrins.

Preferred examples of plant, animal, and microbial extracts include iris extract, *Angelica keiskei* extract, *Thujopsis dolabrata* extract, asparagus extract, avocado extract, *Hydrangea serrata* leaf extract, almond extract, *Althea officinalis* root extract, *Arnica montana* extract, aloe extract, apricot extract, apricot kernel extract, ginkgo extract, *Artemisia capillaris* flower extract, fennel fruit extract, turmeric root extract, oolong tea extract, *uva-ursi* extract, rose fruit extract, *Echinacea angustifolia* leaf extract, *Isodonis japonicus* extract, *Scutellaria* root extract, *Phellodendron* bark extract, *Coptis* rhizome extract, barley extract, *Panax ginseng* extract, *Hypericum perforatum* extract, *Lamium album* extract, *Ononis spinosa* extract, *Nasturtium officinale* extract, orange extract, dried sea water residues, seaweed extract, Japanese persimmon leaf extract, *Pyracantha fortuneana* fruit extract, hydrolyzed elastin, hydrolyzed wheat powder, hydrolyzed silk, *Pueraria* root extract, *Chamomilla recutita* extract, oil-soluble *Chamomilla recutita* extract, carrot extract, *Artemisia capillaris* extract, *Avena fatua* extract, *Hibiscus sabdariffa* extract, licorice extract, oil-soluble licorice extract, kiwi fruit extract, kiou extract, *Auricularia auricula-judae* extract, *Cinchona* bark extract, cucumber extract, *Paulownia tomentosa* leaf extract, guanosine, guava extract, *Sophora* root extract, *Gardenia jasminoides* extract, *Sasa veitchii* extract, *Sophoraflavescens* extract, walnut extract, chestnut extract, grapefruit extract, *Clematis vitalba* extract, black rice extract, black sugar extract, black vinegar, chlorella extract, mulberry extract, gentian extract, geranium herb extract, black tea extract, yeast extract, magnolia bark extract, coffee extract, burdock root extract, rice extract, fermented rice extract, fermented rice bran extract, rice germ oil, comfrey extract, collagen, bilberry extract, *Asiasarum* root extract, *Bupleurum* root extract, umbilical cord extract, saffron extract, salvia extract, *Saponaria officinalis* extract, bamboo grass extract, *Crataegus cuneata* fruit extract, *Bombyx mori excrementum* extract, *Zanthoxylum piperitum* peel extract, shiitake mushroom extract, *Rehmannia glutinosa* root extract, *Lithospermum* root extract, *Perilla frutescens* extract, *Tilia japonica* extract, *Filipendula multijuga* extract, jatoba extract, peony root extract, ginger extract, *Acorus calamus* root extract, *Betula alba* extract, *Tremella fuciformis* extract, *Equisetum arvense* extract, stevia extract, stevia fermentation product, *Tamarix chinensis* extract, *Hedera helix* extract, *Crataegus oxycantha* extract, *Sambucus nigra* extract, *Achillea millefolium* extract, *Mentha piperita* extract, sage extract, mallow extract, *Cnidium rhizome* extract, swertia herb extract, mulberry bark extract, rhubarb extract, soybean extract, jujubi extract, thyme extract, dandelion extract, lichens extract, tea extract, clove extract, *Imperata cylindrica* extract, *Citrus unshiu* peel extract, tea tree oil, *Rubus suavissimus* extract, capsicum extract, *Angelica acutiloba* root extract, *Calendula officinalis* extract, peach kernel extract, bitter orange peel extract, *Houttuynia cordata* extract, tomato extract, natto extract, carrot extract, garlic extract, *Rosa canina* fruit extract, hibiscus extract, *Ophiopogon* tuber extract, lotus extract, parsley extract, birch extract, honey, *Hamamelis virginiana* extract, *Parietaria officinalis* extract, *Rabdosia japonica* extract, bisabolol, cypress extract, *Bifidobacterium* extract, *Eriobotrya japonica* extract, *Tussilago faifara* extract, Japanese butterbur flower-bud extract, *Poria cocos sclerotium* extract, butcher's broom extract, grape extract, grape seed extract, propolis, *Luffa cylindrica* extract, safflower extract, peppermint extract, *Tilia miqueliaria* extract, *Paeonia suffruticosa* extract, hop extract, *Rosa rugosa* extract, pine extract, *Aesculus hippocastanum* extract, *Lysichiton camtschatcense* extract, *Sapindus mukurossi* extract, *Melissa officinalis* extract, *Nemacystus decipiens* extract, peach extract, cornflower extract, *Eucalyptus globulus* extract, *Saxifraga stolonifera* extract, *Citrus junos* extract, lily extract, coix seed extract, *Artemisia princeps* extract, lavender extract, green tea extract, egg shell membrane extract, apple extract, rooibos tea extract, *Litchi chinensis* extract, lettuce extract, lemon extract, *Forsythia* fruit extract, *Astragalus sinicus* extract, rose extract, rosemary extract, *Anthemis nobilis* extract, royal jelly extract, and *Sanguisorba officinalis* extract.

Examples of antipruritics include diphenhydramine hydrochloride, chlorpheniramine maleate, camphor, and substance P inhibitors.

Examples of exfoliates/keratolytic agents include salicylic acid, sulfur, resorcin, selenium sulfide, and pyridoxine.

Examples of antiperspirants include aluminum chlorohydrate, aluminum chloride, zinc oxide, and zinc p-phenolsulfonate.

Examples of refrigerants include menthol and methyl salicylate.

Examples of astringents include citric acid, tartaric acid, lactic acid, aluminum potassium sulfate, and tannic acid.

Examples of enzymes include superoxide dismutase, catalase, lysozyme chloride, lipase, papain, pancreatin, and protease.

Preferred examples of nucleic acids include ribonucleic acids and salts thereof, deoxyribonucleic acids and salts thereof, and adenosine triphosphate disodium.

Preferred examples of perfumes include synthetic and natural perfumes as well as various perfume blends, such as acetyl cedrene, amylcinnamaldehyde, allylamyl glycolate, β-ionone, Iso E Super, isobutylquinoline, iris oil, irone, indole, ylang-ylang oil, undecanal, undecenal, γ-undecalactone, estragole, eugenol, oakmoss, opoponax resinoid, orange oil, eugenol, aurantiol, galaxolide, carvacrol, L-carvone, camphor, canon, carrot seed oil, clove oil, methyl cinnamate, geraniol, geranyl nitrile, isobornyl acetate, geranyl acetate, dimethylbenzylcarbinyl acetate, styralyl acetate, cedryl acetate, terpinyl acetate, p-t-butylcyclohexyl acetate, vetiveryl acetate, benzyl acetate, linalyl acetate, isopentyl salicylate, benzyl salicylate, sandalwood oil, santalol, cyclamen aldehyde, cyclopentadecanolide, methyl dihydrojasmonate, dihydromyrcenol, jasmine absolute, jasmine lactone, cis-jasmone, citral, citronellol, citronellal, cinnamon bark oil, 1,8-cineole, cinnamaldehyde, styrax resinoid, cedarwood oil, cedrene, cedrol, celery seed oil, thyme oil, damascone, damascenone, thymol, tuberose absolute, decanal, decalactone, terpineol, γ-terpinen, triplal, nerol, nonanal, 2,6-nonadienal, nonalactone, patchouli alcohol, vanilla absolute, vanillin, basil oil, patchouli oil, hydroxycitronellal, α-pinene, piperitone, phenethyl alcohol, phenylacetaldehyde, petitgrain oil, hexylcinnamaldehyde, cis-3-hexenol, Peru balsam, vetiver oil, vetiverol, peppermint oil, pepper oil, heliotropin, bergamot oil, benzyl benzoate, borneol, myrrh resinoid, musk ketone, methylnonylacetaldehyde, γ-methylionone, menthol, L-menthol, L-menthone, eucalyptus oil, β-ionone, lime oil, lavender oil, D-limonene, linalool, lyral, lilial, lemon oil, rose absolute, rose oxide, rose oil, rosemary oil, and various essential oils.

Preferred examples of colors, coloring agents, dyes, and pigments include certified colors such as Brown No. 201, Black No. 401, Violet No. 201, Violet No. 401, Blue No. 1, Blue No. 2, Blue No. 201, Blue No. 202, Blue No. 203, Blue No. 204, Blue No. 205, Blue No. 403, Blue No. 404, Green No. 201, Green No. 202, Green. No. 204, Green No. 205, Green No. 3, Green No. 401, Green No. 402, Red No. 102, Red No. 104-1, Red No. 105-1, Red No. 106, Red No. 2, Red No. 201, Red No. 202, Red No. 203, Red No. 204, Red No. 205, Red No. 206, Red No. 207, Red No. 208, Red No. 213, Red No. 214, Red No. 215, Red No. 218, Red No. 219, Red No. 220, Red No. 221, Red No. 223, Red No. 225, Red No. 226, Red No. 227, Red No. 228, Red No. 230-1, Red No. 230-2, Red No. 231, Red No. 232, Red No. 3, Red No. 401, Red No. 404, Red No. 405, Red No. 501, Red No. 502, Red No. 503, Red No. 504, Red No. 505, Red No. 506, Orange No. 201, Orange No. 203, Orange No. 204, Orange No. 205, Orange No. 206, Orange No. 207, Orange No. 401, Orange No. 402, Orange No. 403, Yellow No. 201, Yellow No. 202-1, Yellow No. 202-2, Yellow No. 203, Yellow No. 204, Yellow No. 205, Yellow No. 4, Yellow No. 401, Yellow No. 402, Yellow No. 403-1, Yellow No. 404, Yellow No. 405, Yellow No. 406, Yellow No. 407, and Yellow No. 5; other acid dyes such as Acid Red 14; basic dyes such as Arianor Sienna Brown, Arianor Madder Red, Arianor Steel Blue, and Arianor Straw Yellow; nitro dyes such as HC Yellow 2, HC Yellow 5, HC Red 3, 4-hydroxypropylamino-3-nitrophenol, N,N'-bis(2-hydroxyethyl)-2-nitro-p-phenylenediamine, HC Blue 2, and Basic Blue 26; disperse dyes; inorganic white pigments such as titanium dioxide and zinc oxide; inorganic red pigments such as iron oxide (red iron oxide) and iron titanate; inorganic brown pigments such as γ-iron oxide; inorganic yellow pigments such as yellow iron oxide and ocher; inorganic black pigments such as black iron oxide and low-order titanium oxide; inorganic violet pigments such as mango violet and cobalt violet; inorganic green pigments such as chromium oxide, chromium hydroxide, and cobalt titanate; inorganic blue pigments such as ultramarine and prussian blue; pearl pigments such as titanium oxide-coated mica, titanium oxide-coated bismuth oxychloride, titanium oxide-coated talc, colored titanium oxide-coated mica, bismuth oxychloride, and fish scale foil; metal powder pigments such as aluminum powder, copper powder, and gold; surface-treated inorganic and metal powder pigments; organic pigments such as zirconium lake, barium lake, and aluminum lake; surface-treated organic pigments; natural colors and dyes, for example, anthraquinones such as astaxanthin and alizarin, naphthoquinones such as anthocyanidin, β-carotene, catenal, capsanthin, chalcone, carthamin, quercetin, crocin, chlorophyll, curcumin, cochineal, and shikonin, bixin, flavones, betacyanidine, henna, hemoglobin, lycopene, riboflavin, and rutin; oxidation dye intermediates and couplers such as p-phenylenediamine, toluene-2,5-diamine, o-, m-, and p-aminophenols, m-phenylenediamine, 5-amino-2-methylphenol, resorcin, 1-naphthol, and 2,6-diaminopyridine, as well as salts thereof; autoxidation dyes such as indoline; and dihydroxyacetone.

Preferred examples of antiphlogistics and anti-inflammatory agents include glycyrrhizic acid and derivatives thereof, glycyrrhetic acid derivatives, salicylic acid derivatives, hinokitiol, guaiazulene, allantoin, indomethacin, ketoprofen, ibuprofen, diclofenac, loxoprofen, celecoxib, infliximab, etanercept, zinc oxide, hydrocortisone acetate, prednisone, diphenhydramine hydrochloride, and chlorpheniramine maleate; and plant extracts such as peach leaf extract and *Artemisia princeps* leaf extract.

Preferred examples of anti-asthmatic agents, anti-chronic obstructive pulmonary disease agents, anti-allergic agents, and immunomodulators include aminophylline, theophyllines, steroids (such as fluticasone and beclomethasone), leukotriene antagonists, thromboxane inhibitors, Intal, β2 agonists (such as formoterol, salmeterol, albuterol, tulobuterol, clenbuterol, and epinephrine), tiotropium, ipratropium, dextromethorphan, dimemorfan, bromhexine, tranilast, ketotifen, azelastine, cetirizine, chlorpheniramine, mequitazine, tacrolimus, ciclosporin, sirolimus, methotrexate, cytokine modulators, interferon, omalizumab, and protein/antibody preparations.

Preferred examples of anti-infective agents and antifungal agents include oseltamivir, zanamivir, and itraconazole. In addition to these ingredients, the solid base material for external use on skin and the aqueous composition may contain known cosmetic ingredients, known pharmaceutical ingredients, and known food ingredients, such as ingredients described in Japanese Standards of Cosmetic Ingredients, Japanese Cosmetic Ingredients Codex, List of Cosmetics Ingredients Japanese Labelling Names issued by the Japan Cosmetic Industry Association, INCI dictionary (The International Cosmetic Ingredient Dictionary and Handbook), Japanese Standards of Quasi-drug Ingredients, Japanese Pharmacopoeia, Japanese Pharmaceutical Excipients, Japan's Specifications and Standards for Food Additives, and other standards, as well as ingredients described in Japanese and foreign patent publications and patent application publications (including Japanese translations of PCT international application publications and re-publications of PCT international publications) classified as International Patent Classification IPC classes A61K7 and A61K8, in known combinations and in known ratios and amounts.

The solid base material for external use on skin may contain various drugs, for example, local anesthetics such as lidocaine hydrochloride or hair growth-promoting agents such as minoxidil mentioned above.

The amount of these drugs to be contained may vary depending on the efficacy of the drug to be contained; for example, the amount of lidocaine hydrochloride or minoxidil mentioned above may be about 0.1 to 20% by mass, for example, and preferably about 0.5 to 10% by mass, based on the total mass of the obtained solid base material for external use on skin.

### [Method for Producing Solid Base Material for External Use on Skin (1)]

The solid base material for external use on skin can be produced using a lipid peptide compound comprising at least one of compounds of formulas (1) to (3) or pharmacologically usable salts thereof, a surfactant and water, an alkyl alcohol as a thermal stabilizer, and a 1,2-alkanediol or 1,3-alkanediol, as well as optionally a fatty acid, a polyhydric alcohol, an oleaginous base, an organic acid, and other additives, which are mixed with stirring while heating, and then allowed to cool. As described below, the aqueous composition can be produced during this production process.

For example, the solid base material for external use on skin is produced by the following steps:
a) mixing the lipid peptide compound, a surfactant, water, and an alkyl alcohol, and heating the mixture to prepare a solution or dispersion;
b) adding the solution or dispersion to water, and heating the mixture to a temperature not lower than room temperature and lower than 100°C; and
c) cooling the mixture, with stirring, to a temperature lower than the temperature in the above-described heating step, and then allowing the mixture to cool, thereby forming a gel solid (solid base material for external use on skin).

The 1,2-alkanediol or 1,3-alkanediol, the fatty acid, the polyhydric alcohol, the oleaginous base, the organic acid, and other additives may be added in the step of preparing the solution or dispersion in step a), or may be added in advance to the water to which the solution or dispersion is to be added in step b).

The amount of water is preferably 20% by mass or more and less than 95% by mass, based on the total mass of the obtained solid base material for external use on skin.

Furthermore, the amount of water is preferably 30% by mass or more and less than 80% by mass, based on the total mass of the obtained solution or dispersion.

In steps a) and b), the heating temperature is preferably 50 to 90°C, and more preferably 60 to 90°C, for example, 80°C. Stirring is preferably performed while heating. The heating and stirring time in each of these steps varies depending on the types and amounts of the lipid peptide compound and other ingredients such as a surfactant used; typically, these ingredients can be dissolved or dispersed in about 5 to 50 minutes.

Subsequent to steps a) and b), the mixture is cooled with stirring until the liquid temperature of the mixture becomes lower than the temperature in step b) (step c)). The cooling temperature is, for example, from room temperature to about 80°C, from room temperature to about 60°C, or from room temperature to about 40°C.

### [Methods for Producing Aqueous Composition and Solid Base Material for External Use on Skin (2)]

The method for producing the solid base material for external use on skin using the aqueous composition will be hereinafter described.

### <Method for Producing Aqueous Composition>

To produce the aqueous composition, initially, a lipid peptide compound comprising at least one of compounds of formulas (1) to (3) or pharmacologically usable salts thereof, a surfactant, water, and an alkyl alcohol are mixed, and the mixture is heated to prepare a solution or dispersion. During the preparation of the solution or dispersion, a 1,2-alkanediol or 1,3-alkanediol may be optionally added, and a fatty acid, a polyhydric alcohol, an oleaginous base, an organic acid, and other additives may also be added, as appropriate.

This solution or dispersion is then cooled. As a result, the aqueous composition can be obtained.

The heating temperature is preferably 50 to 90°C, and more preferably 60 to 90°C, for example, 80°C. Stirring is preferably performed during heating. The heating (stirring) time varies depending on the types and amounts of the lipid peptide compound and other ingredients such as a surfactant used; typically, the heating (stirring) time is from about 5 to 50 minutes. As a result, a solution or dispersion in which the mixed ingredients are dissolved or dispersed is obtained.

It is preferred to cool, with stirring, the obtained solution or dispersion to a temperature lower than the heating temperature, for example, from room temperature to about 80°C, from room temperature to about 60°C, or from room temperature to about 40°C, and then stop the stirring and allow the solution or dispersion to stand.

The amount of water is preferably 30% by mass or more and less than 80% by mass, based on the total mass of the obtained aqueous composition.

The aqueous composition thus obtained is useful as a premix for preparing the solid base material for external use on skin. As described below, the composition can contain water and other ingredients such as an active ingredient, and thus the solid base material for external use on skin can be readily prepared.

### [Method for Producing Solid Base Material for External Use on Skin Using Aqueous Composition]

The solid base material for external use on skin can be prepared simultaneously with the preparation of the aqueous composition, i.e., through the following steps 1) to 4):
1) a heating step of heating a mixture system to a temperature not lower than room temperature and lower than 100°C, wherein the mixture system comprises a surfactant and water, and a lipid peptide compound comprising at least one of compounds of formulas (1) to (3) or pharmacologically usable salts thereof;
2) a preparation step of adding at least one saturated or unsaturated monohydric alcohol having a carbon atom number of 8 to 30 to the heated mixture system to prepare the aqueous composition;
3) a mixing step of adding the prepared aqueous composition to an aqueous phase heated to a temperature not lower than room temperature and lower than 100°C, followed by mixing, or adding an aqueous phase heated to a temperature not lower than room temperature and lower than 100°C to the prepared aqueous composition, followed by mixing; and
4) a cooling step of cooling the mixture obtained in the mixing step to form a gel.

The aqueous phase contains water, may further contain a polyhydric alcohol and an oleaginous base, and may additionally contain a 1,2-alkanediol or 1,3-alkanediol, an alkyl alcohol, a fatty acid, an organic acid, and other additives.

Furthermore, in the mixing step, a solution of a drug may be further added to produce a solid base material (preparation) for external use on skin containing the drug.

The heating temperature for the mixture system (and the aqueous composition) in step 1 (and step 2)) is preferably 50 to 90°C, and more preferably 60 to 90°C, for example, 70 or 80°C. These steps are preferably performed with stirring. The heating (stirring) time in each of these steps varies depending on the types and amounts of the lipid peptide compound and other ingredients such as a surfactant contained in the aqueous composition; typically, the heating time is from about 5 to 50 minutes. Through these steps, the aqueous composition is homogeneously dissolved.

The heating temperature for the aqueous phase in step 3) is preferably 50 to 90°C, and more preferably 60 to 90°C, for example, 70 or 80°C. In particular, when the aqueous phase contains other ingredients such as an oleaginous base, the aqueous phase is preferably heated with stirring. Furthermore, when the aqueous phase contains a polyhydric alcohol and an oleaginous base, as well as a 1,2-alkanediol or 1,3-alkanediol, an alkyl alcohol, a fatty acid, and other additives, the aqueous phase is preferably heated (with stirring) typically for about 5 to 50 minutes, until these ingredients are homogeneously dissolved or dispersed. The heating temperature for the aqueous phase may be the same as the heating temperature for the aqueous composition.

Subsequently in step 4), the mixture obtained in the preceding step is cooled to form a gel. In this case, the mixture may be cooled with stirring. When the mixture is cooled with stirring, it is preferred to perform the stirring until the cooling temperature reaches, for example, room temperature to 80°C or room temperature to 60°C, for example, about 60°C, and then stop the stirring and allow the mixture to cool. It is particularly preferred to stop the stirring at 50°C or lower, and allow the mixture to cool.

Likewise, in the solid base material for external use on skin thus obtained using the aqueous composition, the amount of water to be contained therein is preferably 20% by mass or more and less than 95% by mass, based on the total mass of the solid base material for external use on skin.

### Examples

The present invention will be hereinafter described in detail with reference to examples and test examples; however, the present invention is not limited to these examples.

### [Synthesis Example 1: Synthesis of Lipid Peptide (N-Palmitoyl-Gly-His)]

The lipid peptide compound used in the examples was synthesized in accordance with the following method.

A500-mL four-necked flask was charged with 14.2 g (91.6 mmol) of histidine, 30.0 g (91.6 mmol) of N-palmitoyl-Gly-methyl, and 300 g of toluene, and then 35.3 g (183.2 mmol) of a 28% methanol solution of sodium methoxide as a base was added thereto. The mixture was heated to 60°C in an oil bath, and continuously stirred for 1 hour. The oil bath was then removed, and the solution was allowed to cool to 25°C. To the solution was added 600 g of acetone to cause reprecipitation, and the precipitate was collected by filtration. The obtained solid was dissolved in a mixed solution of 600 g of water and 750 g of methanol. To the solution was added 30.5 ml (183.2 mmol) of 6N hydrochloric acid to neutralize the solution and precipitate a solid, and the solid was collected by filtration. Next, the obtained solid was dissolved in a mixed solution of 120 g of tetrahydrofuran and 30 g of water at 60°C, and then 150 g of ethyl acetate was added thereto. The mixture was cooled from 60 to 30°C. The precipitated solid was then collected by filtration. Furthermore, the obtained solid was dissolved in a solvent of 120 g of tetrahydrofuran and 60 g of acetonitrile. The solution was heated to 60°C, stirred for 1 hour, and then cooled. The solid was then collected by filtration. The obtained solid was washed with 120 g of water, collected by filtration, and then dried under reduced pressure to give 26.9 g of the free form of N-palmitoyl-Gly-His (hereinafter also simply referred to as Pal-GH) as white crystals (yield 65%).

### [Examples 1 to 27, and Comparative Examples 1 to 3]

### <Method for Preparing Samples>

In accordance with each of the formulations shown in Tables 1 to 3, all the ingredients were weighed out in a beaker. The beaker was heated to a temperature around 80°C to homogeneously dissolve all the ingredients. A sample tube No. 7 was charged with a suitable amount of the obtained solution, sealed with a cap, and allowed to cool at room temperature to prepare a sample in solid form.

### <Method for Evaluation in Thermal Stability Test>

A thermostat capable of adjusting the temperature (product name: JUNIOR from Kusumoto Chemicals, Ltd.) was set at 40°C, and the sample after being allowed to cool as described above was stored for 6 hours in the thermostat. After the storage, the condition of the sample was visually examined. The sample was evaluated as (○) when it remained solid, evaluated as (Δ) when the side surface of the sample dissolved with heat, or evaluated as (×) when the sample completely dissolved into a solution with heat.

After the evaluation, the sample was returned into the thermostat, and the temperature within the thermostat was increased to 45°C. The sample was further stored for 6 hours in the thermostat, and then the condition of the sample was evaluated in the same manner as described above. The sample was then returned into the thermostat, and the temperature within the thermostat was increased in increments of 5°C. The sample was stored for 6 hours in the thermostat, and then the condition of the sample was evaluated. This procedure was repeated at a set temperature of 50, 55, 60, or 65°C. The results of evaluation at the respective temperatures are summarized in Tables 1 to 3 below.

**Table 1**

| Components (g) | | Examples | | | | | | | | | Comparative Example |
|---|---|---|---|---|---|---|---|---|---|---|---|
| | | 1 | 2 | 3 | 4 | 5 | 6 | 7 | 8 | 9 | 1 |
| Pal-GH | | 5.0 | | | | | | | | | 5.0 |
| 1,2-Hexanediol^{*1} | | 2.0 | | | | | | | | | 2.0 |
| Polyoxyethylene Lauryl Ether^{*3} | | 4.0 | | | | | | | | | 4.0 |
| Cetanol^{*4} | | 0.5 | 1.0 | 2.0 | | | | | | | |
| Stearyl Alcohol^{*5} | | | | | 0.5 | 1.0 | 2.0 | | | | |
| Behenyl Alcohol^{*6} | | | | | | | | 0.5 | 1.0 | 2.0 | |
| Purified Water | | 88.5 | 88.0 | 87.0 | 88.5 | 88.0 | 87.0 | 88.5 | 88.0 | 87.0 | 89.0 |
| Total | | 100 | 100 | 100 | 100 | 100 | 100 | 100 | 100 | 100 | 100 |
| Stick Shape after Six Hours of Storage | Stored at 40°C | ○ | ○ | ○ | ○ | ○ | ○ | ○ | ○ | ○ | ○ |
| | Stored at 45°C | ○ | ○ | ○ | ○ | ○ | ○ | ○ | ○ | ○ | Δ |
| | Stored at 50°C | ○ | ○ | ○ | ○ | ○ | ○ | ○ | ○ | ○ | × |
| | Stored at 55°C | Δ | ○ | ○ | × | ○ | ○ | × | ○ | ○ | × |
| | Stored at 60°C | × | Δ | ○ | × | Δ | Δ | × | × | × | × |
| | Stored at 65°C | × | × | × | × | × | × | × | × | × | × |

| | | | | | | | | | | | |
|---|---|---|---|---|---|---|---|---|---|---|---|
| *1: from ITO Inc. *3: from Nikko Chemicals Co., Ltd. *4: from Tokyo Chemical Industry Co., Ltd. *5: from Tokyo Chemical Industry Co., Ltd. *6: from Tokyo Chemical Industry Co., Ltd. | | | | | | | | | | | |

**Table 2**

| Components (g) | | Examples | | | | | | | | | Comparative Example |
|---|---|---|---|---|---|---|---|---|---|---|---|
| | | 10 | 11 | 12 | 13 | 14 | 15 | 16 | 17 | 18 | 2 |
| Pal-GH | | 5.0 | | | | | | | | | 5.0 |
| 2-Ethyl-1,3-Hexanediol^{*2} | | 2.0 | | | | | | | | | 2.0 |
| Polyoxyethylene Lauryl Ether^{*3} | | 4.0 | | | | | | | | | 4.0 |
| Cetanol^{*4} | | 0.5 | 1.0 | 2.0 | | | | | | | |
| Stearyl Alcohol^{*5} | | | | | 0.5 | 1.0 | 2.0 | | | | |
| Behenyl Alcohol^{*6} | | | | | | | | 0.5 | 1.0 | 2.0 | |
| Purified Water | | 88.5 | 88.0 | 87.0 | 88.5 | 88.0 | 87.0 | 88.5 | 88.0 | 87.0 | 89.0 |
| Total | | 100 | 100 | 100 | 100 | 100 | 100 | 100 | 100 | 100 | 100 |
| Stick Shape after Six Hours of Storage | Stored at 40°C | ○ | ○ | ○ | ○ | ○ | ○ | ○ | ○ | ○ | ○ |
| | Stored at 45°C | ○ | ○ | ○ | ○ | ○ | ○ | ○ | ○ | ○ | Δ |
| | Stored at 50°C | ○ | ○ | ○ | ○ | ○ | ○ | ○ | ○ | ○ | × |
| | Stored at 55°C | × | ○ | ○ | × | ○ | ○ | × | ○ | ○ | × |
| | Stored at 60°C | × | Δ | Δ | × | Δ | Δ | × | × | × | × |
| | Stored at 65°C | × | × | × | × | × | × | × | × | × | × |

| | | | | | | | | | | | |
|---|---|---|---|---|---|---|---|---|---|---|---|
| *2: from KH Neochem Co., Ltd. *3: from Nikko Chemicals Co., Ltd. *4: from Tokyo Chemical Industry Co., Ltd. *5: from Tokyo Chemical Industry Co., Ltd. *6: from Tokyo Chemical Industry Co., Ltd. | | | | | | | | | | | |

**Table 3**

| Components (g) | | Examples | | | | | | | | | Comparative Example |
|---|---|---|---|---|---|---|---|---|---|---|---|
| | | 19 | 20 | 21 | 22 | 23 | 24 | 25 | 26 | 27 | 3 |
| Pal-GH | | 5.0 | | | | | | | | | 5.0 |
| 1,2-Hexanediol^{*1} | | 1.0 | | | | | | | | | 1.0 |
| Decaglyceryl Diisostearate ^{*7} | | 0.75 | | | | | | | | | 0.75 |
| Cetanol*⁴ | | 0.5 | 1.0 | 2.0 | | | | | | | |
| Stearyl Alcohol^{*5} | | | | | 0.5 | 1.0 | 2.0 | | | | |
| Behenyl Alcohol^{*6} | | | | | | | | 0.5 | 1.0 | 2.0 | |
| Purified Water | | 92.75 | 92.25 | 91.25 | 92.75 | 92.25 | 91.25 | 92.75 | 92.25 | 91.25 | 93.25 |
| Total | | 100 | 100 | 100 | 100 | 100 | 100 | 100 | 100 | 100 | 100 |
| Stick Shape after Six Hours of Storage | Stored at 40°C | ○ | ○ | ○ | ○ | ○ | ○ | ○ | ○ | ○ | ○ |
| | Stored at 45°C | ○ | ○ | ○ | ○ | ○ | ○ | ○ | ○ | ○ | ○ |
| | Stored at 50°C | ○ | ○ | ○ | ○ | ○ | ○ | ○ | ○ | ○ | × |
| | Stored at 55°C | Δ | ○ | ○ | Δ | ○ | ○ | Δ | ○ | ○ | × |
| | Stored at 60°C | Δ | Δ | ○ | × | Δ | Δ | × | × | Δ | × |
| | Stored at 65°C | × | × | Δ | × | × | × | × | × | × | × |

| | | | | | | | | | | | |
|---|---|---|---|---|---|---|---|---|---|---|---|
| *1: from ITO Inc. *4: from Tokyo Chemical Industry Co., Ltd. *5: from Tokyo Chemical Industry Co., Ltd. *6: from Tokyo Chemical Industry Co., Ltd. *7: from Nikko Chemicals Co., Ltd | | | | | | | | | | | |

As shown in the tables above, the samples according to Examples 1 to 27 stably remained in the solid state under storage conditions at 50°C, which serve as a criterion of thermal stability. Furthermore, the thermal stability tended to increase as the amount of the alkyl alcohol contained in the sample increased.

In contrast, the samples according to Comparative Examples 1 to 3 not containing an alkyl alcohol turned into a solution at 50°C, and did not exhibit thermal stability.

### [Examples 28 to 30 and Comparative Example 4]

The ingredients of Phase A were weighed out in the proportions shown in Table 4 below (total amount: 25 g). The weighed-out Phase A was heated to 80°C to homogeneously dissolve all the ingredients. Each type of alkyl alcohol of Phase B was added in an amount of 1 g to Phase A kept at 80°C, and dissolved. Then, Phase C (purified water) previously heated to 80°C was gradually added to Phase A for dilution such that the total amount of the mixture became 100 g. The mixture was stirred to homogeneity. A screw tube No. 7 (from Maruemu Corporation) was charged with 40 g of the obtained solution, sealed with a cap, and allowed to cool at room temperature to give a stick-shaped solid base material.

In Comparative Example 4, a stick-shaped solid base material was obtained as in Examples 28 to 30, except that Phase B was not added.

### <Method for Evaluation in Thermal Stability Test>

A thermostat capable of adjusting the temperature (product name: JUNIOR from Kusumoto Chemicals, Ltd.) was set at 50°C, and each of the stick-shaped solid base materials obtained in Examples 28 to 30 and Comparative Example 4 placed in the screw tube was stored for 1 month in the thermostat. After the storage, the condition of each of the base materials was visually examined. When the properties of the base material remained unchanged from the properties before the storage (the base material remained solid), then the base material was evaluated as (○), i.e., having thermal stability. When the base material dissolved into a solution, then the base material was evaluated as (×), i.e., not having thermal stability. The results are summarized in Table 4.

**Table 4**

| Components (g) | | | Example 28 | Example 29 | Example 30 | Comparative Example 4 |
|---|---|---|---|---|---|---|
| Phase A | Pal-GH | 20 wt% | 5.0 | 5.0 | 5.0 | 5.0 |
| | Polyoxyethylene Lauryl Ether^{*3} | 16 wt% | 4.0 | 4.0 | 4.0 | 4.0 |
| | 2-Ethyl-1,3-Hexanediol^{*2} | 8 wt% | 2.0 | 2.0 | 2.0 | 2.0 |
| | Propylene Glycol^{*8} | 20 wt% | 5.0 | 5.0 | 5.0 | 5.0 |
| | Stearic Acid^{*9} | 1 wt% | 0.25 | 0.25 | 0.25 | 0.25 |
| | Purified Water | 35 wt% | 8.75 | 8.75 | 8.75 | 8.75 |
| | Phase A, Total | 100 wt% | 25 | 25 | 25 | 25 |
| Phase B | Cetanol^{*4} | | 1 | | | |
| | Stearyl Alcohol^{*5} | | | 1 | | |
| | Behenyl Alcohol^{*6} | | | | 1 | |
| Phase C | Purified Water | | 74 | 74 | 74 | 75 |
| All Ingredients, Total | | | 100 | 100 | 100 | 100 |
| Stick Shape after 1-Month of Storage at 50°C | | | ○ | ○ | ○ | × |

| | | | | | | |
|---|---|---|---|---|---|---|
| *2: from KH Neochem Co., Ltd. *3: from Nikko Chemicals Co., Ltd. *4: from Tokyo Chemical Industry Co., Ltd. *5: from Tokyo Chemical Industry Co., Ltd. *6: from Tokyo Chemical Industry Co., Ltd. *8: from Junsei Chemical Co., Ltd. *9: from Kao Corporation [trade name: Lunac S-98] | | | | | | |

As shown in Table 4, the solid base materials according to Examples 28 to 30, each containing an alkyl alcohol, remained in the solid state after 1-month of storage at 50°C. However, the solid base material according to Comparative Example 4 not containing an alkyl alcohol did not remain in the solid state, and turned into a semi-transparent solution. Thus, the addition of a suitable amount of an alkyl alcohol was found to markedly improve the thermal stability of the solid base material.

### [Examples 31 to 33]

The ingredients of Phase A were weighed out in the proportions shown in Table 5 below (total amount: 25 g). The weighed-out Phase A was heated to 80°C to homogeneously dissolve all the ingredients. Lauryl alcohol of Phase B was added in an amount of 1 g to Phase A kept at 80°C, and dissolved. Then, Phase C (a drug-mixture phase in each of Examples 32 and 33) previously heated to 80°C was gradually added to Phase A for dilution such that the total amount of the mixture became 100 g. The mixture was stirred to homogeneity. A screw tube No. 7 (from Maruemu Corporation) was charged with 40 g of the obtained solution, sealed with a cap, and allowed to cool at room temperature to give a solid base material.

### <Method for Evaluation in Thermal Stability Test>

A thermostat capable of adjusting the temperature (product name: JUNIOR from Kusumoto Chemicals, Ltd.) was set at 50°C, and each of the solid base materials obtained in Examples 31 to 33 placed in the screw tube was stored for 1 month in the thermostat. After the storage, the condition of each of the base materials was visually examined. When the properties of the base material remained unchanged from the properties before the storage (the base material remained solid), then the base material was evaluated as (○), i.e., having thermal stability. When the base material dissolved into a solution, then the base material was evaluated as (×), i.e., not having thermal stability. The results are shown in Table 5.

### <Method for Measuring Breaking Strength>

Each of the solid base materials after 1-month storage at 50°C was measured for breaking strength with YAMADEN RHEONER II CREEP METER RE2-33005B (from Yamaden Co., Ltd.), using a measurement rate of 0.5 mm/sec, a measurement distortion factor of 20%, a storing pitch of 0.10 sec, and the jig 30349-3. The results are shown in Table 5.

**Table 5**

| Components (g) | | | Example 31 | Example 32 | Example 33 |
|---|---|---|---|---|---|
| Phase A | Pal-GH | 20 wt% | 5.0 | 5.0 | 5.0 |
| | Polyoxyethylene Lauryl Ether^{*3} | 16 wt% | 4.0 | 4.0 | 4.0 |
| | 2-Ethyl-1,3-Hexanediol^{*2} | 8 wt% | 2.0 | 2.0 | 2.0 |
| | Propylene Glycol^{*8} | 20 wt% | 5.0 | 5.0 | 5.0 |
| | Stearic Acid^{*9} | 1 wt% | 0.25 | 0.25 | 0.25 |
| | Purified Water | 35 wt% | 8.75 | 8.75 | 8.75 |
| | Phase A, Total | 100 wt% | 25 | 25 | 25 |
| Phase B | Cetanol^{*4} | | 1 | 1 | 1 |
| Phase C | Lidocaine Hydrochloride^{*10} | | | 5 | |
| | Minoxidil^{*11} | | | | 5 |
| | Propylene Glycol^{*8} | | 50 | 50 | 50 |
| | Purified Water | | 24 | 19 | 19 |
| All Ingredients, Total | | | 100 | 100 | 100 |
| Stick Shape after 1-Month of Storage at 50°C | | | ○ | ○ | ○ |
| Breaking Strength [× 10⁵ Pa] | | | 1.5 | 1.5 | 1.55 |

| | | | | | |
|---|---|---|---|---|---|
| *2: from KH Neochem Co., Ltd. *3: from Nikko Chemicals Co., Ltd. *4: from Tokyo Chemical Industry Co., Ltd. *8: from Junsei Chemical Co., Ltd. *9: from Kao Corporation [trade name: Lunac S-98] *10: from Aldrich Co. LLC. *11: from Tokyo Chemical Industry Co., Ltd. | | | | | |

As shown in Table 5, the base materials according to Examples 32 and 33, each containing 5% by mass of lidocaine hydrochloride or minoxidil as a drug, also showed no change in properties after 1-month storage at 50°C, and were found to be stable to heat. Furthermore, as a result of the breaking strength measurement, these base materials showed no change in the strength of the stick, as compared to the base material according to Example 31 not containing a drug, and were found to be capable of containing a drug without affecting the hardness or properties of the base material.

## Claims

1. Use of at least one saturated or unsaturated monohydric alcohol having a carbon atom number of 8 to 30 in a stick shaped solid base material for external use on skin comprising:
a surfactant and water;
a lipid peptide compound comprising at least one of compounds of formulas (1) to (3): 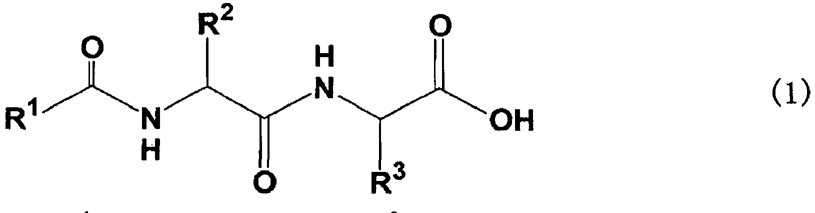 (wherein R¹ is a C₉₋₂₃ aliphatic group; R² is a hydrogen atom or a C₁₋₄ alkyl group optionally having a C₁ or C₂ branched chain; and R³ is a -(CH₂)ₙ-X group, wherein n is a number from 1 to 4, and X is amino group, guanidino group, -CONH₂ group, or a 5-membered ring or 6-membered ring optionally having one to three nitrogen atoms or a fused heterocyclic ring composed of the 5-membered ring and the 6-membered ring); 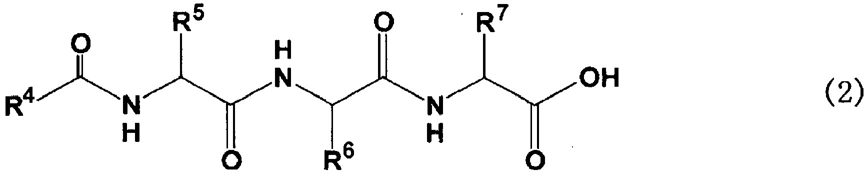 (wherein R⁴ is a C₉₋₂₃ aliphatic group; and R⁵ to R⁷ are each independently a hydrogen atom, a C₁₋₄ alkyl group optionally having a C₁ or C₂ branched chain, or a -(CH₂)ₙ-X group, wherein n is a number from 1 to 4, and X is amino group, guanidino group, -CONH₂ group, or a 5-membered ring or 6-membered ring optionally having one to three nitrogen atoms or a fused heterocyclic ring composed of the 5-membered ring and the 6-membered ring); 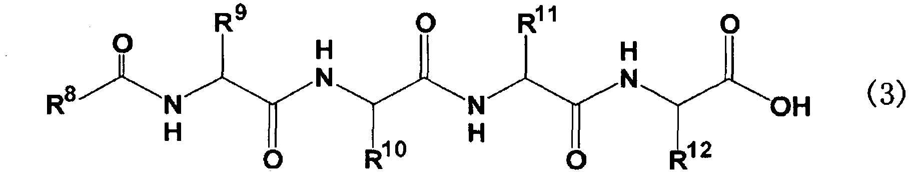 (wherein R⁸ is a C₉₋₂₃ aliphatic group; and R⁹ to R¹² are each independently a hydrogen atom, a C₁₋₄ alkyl group optionally having a C₁ or C₂ branched chain, or a -(CH₂)ₙ-X group, wherein n is a number from 1 to 4, and X is amino group, guanidino group, -CONH₂ group, or a 5-membered ring or 6-membered ring optionally having one to three nitrogen atoms or a fused heterocyclic ring composed of the 5-membered ring and the 6-membered ring); or
pharmacologically usable salts thereof
for increasing the thermal stability of the stick shaped solid base material.

2. Use according to claim 1, wherein the solid base material further comprises a 1,2-alkanediol or 1,3-alkanediol.

3. Use according to claim 1 or 2, wherein the solid base material further comprises at least one fatty acid.

4. Use according to claim 2 or 3, wherein the solid base material further comprises at least one polyhydric alcohol different from the 1,2-alkanediol or 1,3-alkanediol.

5. Use according to any one of claims 1 to 4, wherein the surfactant is one or more compounds selected from the group consisting of ethylene glycol alkyl ethers, phospholipids, polyglycerin fatty acid esters, and polyoxyethylene polyoxypropylene alkyl ethers.

6. Use according to any one of claims 3 to 5, wherein the fatty acid is stearic acid.

7. Use according to any one of claims 4 to 6, wherein the polyhydric alcohol is glycerin, propylene glycol, or polyethylene glycol.

8. Use according to any one of claims 1 to 7, wherein the solid base material further comprises at least one oleaginous base.

9. Use according to any one of claims 1 to 8, wherein the solid base material further comprises at least one organic acid.

10. Use according to any one of claims 1 to 9, wherein the solid base material is used for cosmetics or pharmaceuticals.

11. A method for producing the solid base material for external use on skin as defined in claim 1, comprising:
a heating step of heating a mixture system to a temperature not lower than room temperature and lower than 100°C, wherein the mixture system comprises:
a surfactant and water; and
a lipid peptide compound comprising at least one of compounds of formulas (1) to (3): 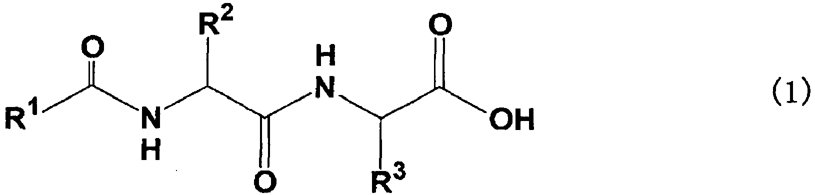 (wherein R¹ is a C₉₋₂₃ aliphatic group; R² is a hydrogen atom or a C₁₋₄ alkyl group optionally having a C₁ or C₂ branched chain; and R³ is a -(CH₂)ₙ-X group, wherein n is a number from 1 to 4, and X is amino group, guanidino group, -CONH₂ group, or a 5-membered ring or 6-membered ring optionally having one to three nitrogen atoms or a fused heterocyclic ring composed of the 5-membered ring and the 6-membered ring); 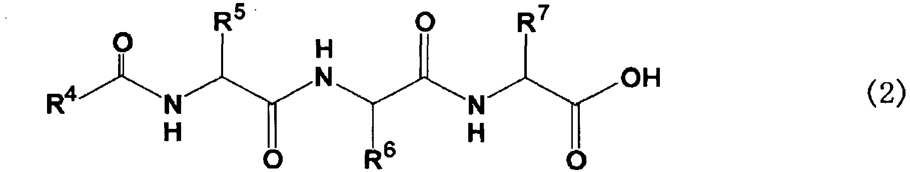 (wherein R⁴ is a C₉₋₂₃ aliphatic group; and R⁵ to R⁷ are each independently a hydrogen atom, a C₁₋₄ alkyl group optionally having a C₁ or C₂ branched chain, or a -(CH₂)ₙ-X group, wherein n is a number from 1 to 4, and X is amino group, guanidino group, -CONH₂ group, or a 5-membered ring or 6-membered ring optionally having one to three nitrogen atoms or a fused heterocyclic ring composed of the 5-membered ring and the 6-membered ring); 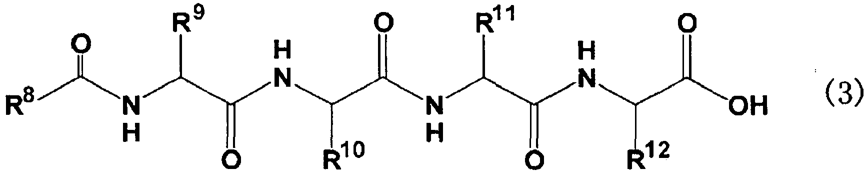 (wherein R⁸ is a C₉₋₂₃ aliphatic group; and R⁹ to R¹² are each independently a hydrogen atom, a C₁₋₄ alkyl group optionally having a C₁ or C₂ branched chain, or a -(CH₂)ₙ-X group, wherein n is a number from 1 to 4, and X is amino group, guanidino group, -CONH₂ group, or a 5-membered ring or 6-membered ring optionally having one to three nitrogen atoms or a fused heterocyclic ring composed of the 5-membered ring and the 6-membered ring); or
pharmacologically usable salts thereof;
a preparation step of adding at least one saturated or unsaturated monohydric alcohol having a carbon atom number of 8 to 30 to the heated mixture system to prepare the aqueous composition according to any one of claims 12 to 17;
a mixing step of adding the prepared aqueous composition to an aqueous phase heated to a temperature not lower than room temperature and lower than 100°C, followed by mixing, or adding an aqueous phase heated to a temperature not lower than room temperature and lower than 100°C to the prepared aqueous composition, followed by mixing; and
a cooling step of cooling the mixture obtained in the mixing step to form a gel.

12. The method according to claim 11, wherein in the mixing step, a solution of a drug is further added.

## Patentansprüche

1. Verwendung von mindestens einem gesättigten oder ungesättigten einwertigen Alkohol mit einer Kohlenstoffatomzahl von 8 bis 30 in einem stabförmigen festen Basismaterial zur äußerlichen Anwendung auf der Haut, umfassend:
ein Tensid und Wasser;
eine Lipidpeptidverbindung, umfassend mindestens eine der Verbindungen der Formeln (1) bis (3): 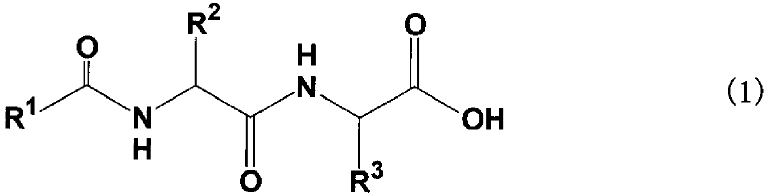 (worin R¹ eine aliphatische C₉₋₂₃-Gruppe ist; R² ein Wasserstoffatom oder eine C₁₋₄-Alkylgruppe, optional mit einer C₁- oder C₂-Verzweigungskette, ist; und R³ eine -(CH₂)ₙX-Gruppe ist, wobei n eine Zahl von 1 bis 4 ist, und X eine Aminogruppe, eine Guanidinogruppe, eine -CONH₂-Gruppe oder ein 5-gliedriger Ring oder ein 6-gliedriger Ring, optional mit ein bis drei Stickstoffatomen, oder ein kondensierter heterocyclischer Ring, zusammengesetzt aus dem 5-gliedrigen Ring und dem 6-gliedrigen Ring, ist); 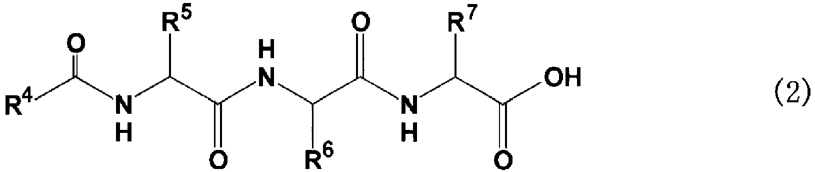 (worin R⁴ eine aliphatische C₉₋₂₃-Gruppe ist; und R⁵ bis R⁷ jeweils unabhängig voneinander ein Wasserstoffatom, eine C₁₋₄-Alkylgruppe, optional mit einer C₁- oder C₂-Verzweigungskette, oder eine -(CH₂)ₙX-Gruppe sind, wobei n eine Zahl von 1 bis 4 ist, und X eine Aminogruppe, eine Guanidinogruppe, eine -CONH₂-Gruppe oder ein 5-gliedriger Ring oder ein 6-gliedriger Ring, optional mit ein bis drei Stickstoffatomen, oder ein kondensierter heterocyclischer Ring, zusammengesetzt aus dem 5-gliedrigen Ring und dem 6-gliedrigen Ring, ist); 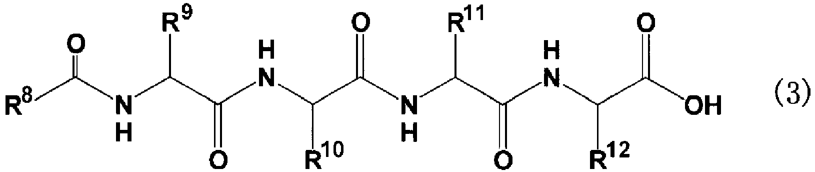 (worin R⁸ eine aliphatische C₉₋₂₃-Gruppe ist; und R⁹ bis R¹² jeweils unabhängig voneinander ein Wasserstoffatom, eine C₁₋₄-Alkylgruppe, optional mit einer C₁- oder C₂-Verzweigungskette, oder eine -(CH₂)ₙX-Gruppe sind, wobei n eine Zahl von 1 bis 4 ist, und X eine Aminogruppe, eine Guanidinogruppe, eine -CONH₂-Gruppe oder ein 5-gliedriger Ring oder ein 6-gliedriger Ring, optional mit ein bis drei Stickstoffatomen, oder ein kondensierter heterocyclischer Ring, zusammengesetzt aus dem 5-gliedrigen Ring und dem 6-gliedrigen Ring, ist); oder
pharmakologisch verwendbare Salze davon
zur Erhöhung der thermischen Stabilität des stabförmigen festen Basismaterials.

2. Verwendung gemäß Anspruch 1, wobei das feste Basismaterial weiterhin ein 1,2-Alkandiol oder 1,3-Alkandiol umfasst.

3. Verwendung gemäß Anspruch 1 oder 2, wobei das feste Basismaterial weiterhin mindestens eine Fettsäure umfasst.

4. Verwendung gemäß Anspruch 2 oder 3, wobei das feste Basismaterial weiterhin mindestens einen mehrwertigen Alkohol umfasst, der von dem 1,2-Alkandiol oder 1,3-Alkandiol verschieden ist.

5. Verwendung gemäß einem der Ansprüche 1 bis 4, wobei das Tensid eine oder mehrere Verbindungen, ausgewählt aus der Gruppe, bestehend aus Ethylenglycolalkylethern, Phospholipiden, Polyglycerinfettsäureestern und Polyoxyethylenpolyoxypropylenalkylethern, ist.

6. Verwendung gemäß einem der Ansprüche 3 bis 5, wobei die Fettsäure Stearinsäure ist.

7. Verwendung gemäß einem der Ansprüche 4 bis 6, wobei der mehrwertige Alkohol Glycerin, Propylenglycol oder Polyethylenglycol ist.

8. Verwendung gemäß einem der Ansprüche 1 bis 7, wobei das feste Basismaterial weiterhin mindestens eine ölige Basis umfasst.

9. Verwendung gemäß einem der Ansprüche 1 bis 8, wobei das feste Basismaterial weiterhin mindestens eine organische Säure umfasst.

10. Verwendung gemäß einem der Ansprüche 1 bis 9, wobei das feste Basismaterial für Kosmetika oder Pharmazeutika verwendet wird.

11. Verfahren zur Herstellung des festen Basismaterials zur äußerlichen Anwendung auf der Haut, wie in Anspruch 1 definiert, umfassend:
einen Erwärmungsschritt des Erwärmens eines Mischungssystems auf eine Temperatur, die nicht niedriger als Raumtemperatur und niedriger als 100°C ist, wobei das Mischungssystem umfasst:
ein Tensid und Wasser; und
eine Lipidpeptidverbindung, umfassend mindestens eine der Verbindungen der Formeln (1) bis (3): 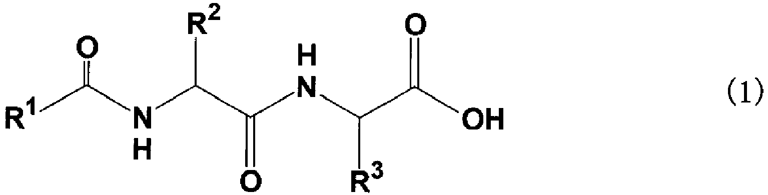 (worin R¹ eine aliphatische C₉₋₂₃-Gruppe ist; R² ein Wasserstoffatom oder eine C₁₋₄-Alkylgruppe, optional mit einer C₁- oder C₂-Verzweigungskette, ist; und R³ eine -(CH₂)ₙX-Gruppe ist, wobei n eine Zahl von 1 bis 4 ist, und X eine Aminogruppe, eine Guanidinogruppe, eine -CONH₂-Gruppe oder ein 5-gliedriger Ring oder ein 6-gliedriger Ring, optional mit ein bis drei Stickstoffatomen, oder ein kondensierter heterocyclischer Ring, zusammengesetzt aus dem 5-gliedrigen Ring und dem 6-gliedrigen Ring, ist); 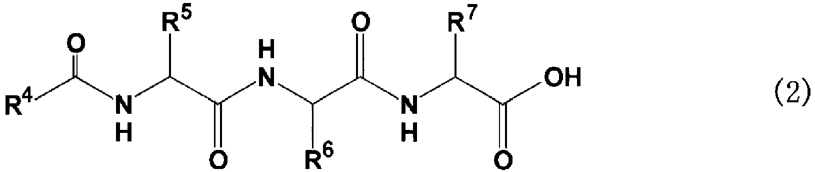 (worin R⁴ eine aliphatische C₉₋₂₃-Gruppe ist; und R⁵ bis R⁷ jeweils unabhängig voneinander ein Wasserstoffatom, eine C₁₋₄-Alkylgruppe, optional mit einer C₁- oder C₂-Verzweigungskette, oder eine -(CH₂)ₙX-Gruppe sind, wobei n eine Zahl von 1 bis 4 ist, und X eine Aminogruppe, eine Guanidinogruppe, eine -CONH₂-Gruppe oder ein 5-gliedriger Ring oder ein 6-gliedriger Ring, optional mit ein bis drei Stickstoffatomen, oder ein kondensierter heterocyclischer Ring, zusammengesetzt aus dem 5-gliedrigen Ring und dem 6-gliedrigen Ring, ist); 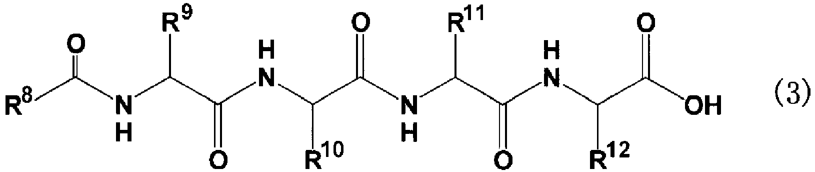 (worin R⁸ eine aliphatische C₉₋₂₃-Gruppe ist; und R⁹ bis R¹² jeweils unabhängig voneinander ein Wasserstoffatom, eine C₁₋₄-Alkylgruppe, optional mit einer C₁- oder C₂-Verzweigungskette, oder eine -(CH₂)ₙX-Gruppe sind, wobei n eine Zahl von 1 bis 4 ist, und X eine Aminogruppe, eine Guanidinogruppe, eine -CONH₂-Gruppe oder ein 5-gliedriger Ring oder ein 6-gliedriger Ring, optional mit ein bis drei Stickstoffatomen, oder ein kondensierter heterocyclischer Ring, zusammengesetzt aus dem 5-gliedrigen Ring und dem 6-gliedrigen Ring, ist); oder
pharmakologisch verwendbare Salze davon;
einen Herstellungsschritt des Hinzufügens von mindestens einem gesättigten oder ungesättigten einwertigen Alkohol mit einer Kohlenstoffatomzahl von 8 bis 30 zu dem erwärmten Mischungssystem, um die wässrige Zusammensetzung gemäß einem der Ansprüche 12 bis 17 herzustellen;
einen Mischschritt des Hinzufügens der hergestellten wässrigen Zusammensetzung zu einer wässrigen Phase, erwärmt auf eine Temperatur, die nicht niedriger als Raumtemperatur und niedriger als 100°C ist, gefolgt von Mischen, oder des Hinzufügens einer wässrigen Phase, erwärmt auf eine Temperatur, die nicht niedriger als Raumtemperatur und niedriger als 100°C ist, zu der hergestellten wässrigen Zusammensetzung, gefolgt von Mischen; und
einen Kühlschritt des Abkühlens der im Mischschritt erhaltenen Mischung, um ein Gel zu bilden.

12. Verfahren gemäß Anspruch 11, wobei in dem Mischschritt zusätzlich eine Lösung eines Wirkstoffs zugegeben wird.

## Revendications

1. Utilisation d'au moins un alcool monohydrique saturé ou insaturé présentant un nombre d'atomes de carbone de 8 à 30 dans un matériau de base solide en forme de bâtonnet pour une utilisation externe sur de la peau comprenant :
un tensioactif et de l'eau ;
un composé peptide lipidique comprenant au moins l'un des composés des formule (1) à (3) : 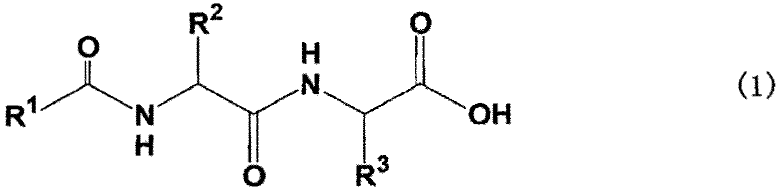 (dans laquelle R¹ est un groupe aliphatique en C₉₋₂₃; R² est un atome d'hydrogène ou un groupe alkyle en C₁₋₄ présentant, facultativement, une chaîne ramifiée en C₁ ou C₂; et R³ est un groupe -(CH₂)ₙ-X, dans laquelle n est un nombre de 1 à 4 et X est un groupe amino, un groupe guanidino, un groupe -CONH₂ ou un cycle à 5 chaînons ou un cycle à 6 chaînons présentant, facultativement, un à trois atomes d'azote ou un cycle hétérocyclique condensé composé du cycle à 5 chaînons et du cycle à 6 chaînons) ; 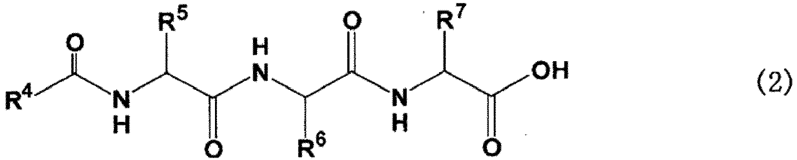 (dans laquelle R⁴ est un groupe aliphatique en C₉₋₂₃; et R⁵ à R⁷ sont chacun indépendamment un atome d'hydrogène, un groupe alkyle en C₁₋₄ présentant, facultativement, une chaîne ramifiée en C₁ ou C₂ ou un groupe -(CH₂)ₙ-X, dans laquelle n est un nombre de 1 à 4 et X est un groupe amino, un groupe guanidino, un groupe -CONH₂ ou un cycle à 5 chaînons ou un cycle à 6 chaînons présentant, facultativement, un à trois atomes d'azote ou un cycle hétérocyclique condensé composé du cycle à 5 chaînons et du cycle à 6 chaînons) ; 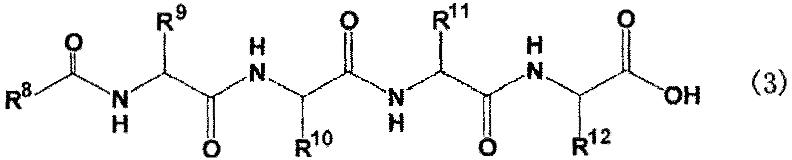 (dans laquelle R⁸ est un groupe aliphatique en C₉₋₂₃ ; et R⁹ à R¹² sont chacun indépendamment un atome d'hydrogène, un groupe alkyle en C₁₋₄ présentant, facultativement, une chaîne ramifiée en C₁ ou C₂ ou un groupe -(CH₂)ₙ-X, dans laquelle n est un nombre de 1 à 4 et X est un groupe amino, un groupe guanidino, un groupe -CONH₂ ou un cycle à 5 chaînons ou un cycle à 6 chaînons présentant, facultativement, un à trois atomes d'azote ou un cycle hétérocyclique condensé composé du cycle à 5 chaînons et du cycle à 6 chaînons) ; ou
des sels pharmaceutiquement utilisables de ceux-ci pour augmenter la stabilité thermique du matériau de base solide en forme de bâtonnet.

2. Utilisation selon la revendication 1, dans laquelle le matériau de base solide comprend en outre un 1,2-alcanediol ou un 1,3-alcanediol.

3. Utilisation selon la revendication 1 ou 2, dans laquelle le matériau de base solide comprend en outre au moins un acide gras.

4. Utilisation selon la revendication 2 ou 3, dans laquelle le matériau de base solide comprend en outre au moins un alcool polyhydrique différent du 1,2-alcanediol ou du 1,3-alcanediol.

5. Utilisation selon l'une quelconque des revendications 1 à 4, dans laquelle le tensioactif est un ou plusieurs composés sélectionnés dans le groupe consistant en les éthers d'alkyle d'éthylène glycol, les phospholipides, les esters d'acide gras de polyglycérine et les éthers d'alkyle de polyoxyéthylène-polyoxypropylène.

6. Utilisation selon l'une quelconque des revendications 3 à 5, dans laquelle l'acide gras est l'acide stéarique.

7. Utilisation selon l'une quelconque des revendications 4 à 6, dans laquelle l'alcool polyhydrique est la glycérine, le propylène glycol ou le polyéthylène glycol.

8. Utilisation selon l'une quelconque des revendications 1 à 7, dans laquelle le matériau de base solide comprend en outre au moins une base oléagineuse.

9. Utilisation selon l'une quelconque des revendications 1 à 8, dans laquelle le matériau de base solide comprend en outre au moins un acide organique.

10. Utilisation selon l'une quelconque des revendications 1 à 9, dans laquelle le matériau de base solide est utilisé pour des produits cosmétiques ou des produits pharmaceutiques.

11. Procédé pour produire le matériau de base solide pour une utilisation externe sur la peau telle que définie dans la revendication 1, comprenant :
une étape de chauffage consistant à chauffer un système de mélange à une température n'étant pas inférieure à la température ambiante et inférieure à 100°C, dans lequel le système de mélange comprend :
un tensioactif et de l'eau ; et
un composé peptide lipidique comprenant au moins l'un des composés des formules (1) à (3) : 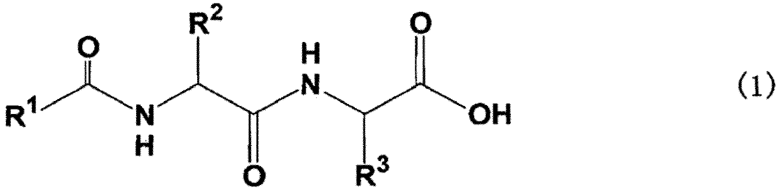 (dans laquelle R¹ est un groupe aliphatique en C₉₋₂₃; R² est un atome d'hydrogène ou un groupe alkyle en C₁₋₄ présentant, facultativement, une chaîne ramifiée en C₁ ou C₂ ; et R³ est un groupe -(CH₂)ₙ-X, dans laquelle n est un nombre de 1 à 4 et X est un groupe amino, un groupe guanidino, un groupe -CONH₂ ou un cycle à 5 chaînons ou un cycle à 6 chaînons présentant, facultativement, un à trois atomes d'azote ou un cycle hétérocyclique condensé composé du cycle à 5 chaînons et du cycle à 6 chaînons) ; 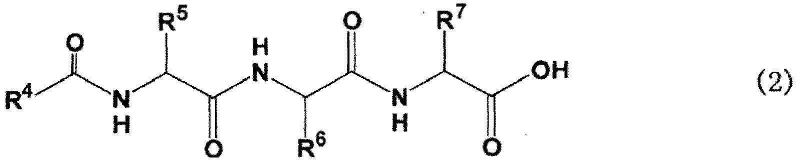 (dans laquelle R⁴ est un groupe aliphatique en C₉₋₂₃; et R⁵ à R⁷ sont chacun indépendamment un atome d'hydrogène, un groupe alkyle en C₁₋₄ présentant, facultativement, une chaîne ramifiée en C₁ ou C₂ ou un groupe -(CH₂)ₙ-X, dans laquelle n est un nombre de 1 à 4 et X est un groupe amino, un groupe guanidino, un groupe -CONH₂ ou un cycle à 5 chaînons ou un cycle à 6 chaînons présentant, facultativement, un à trois atomes d'azote ou un cycle hétérocyclique condensé composé du cycle à 5 chaînons et du cycle à 6 chaînons) ; 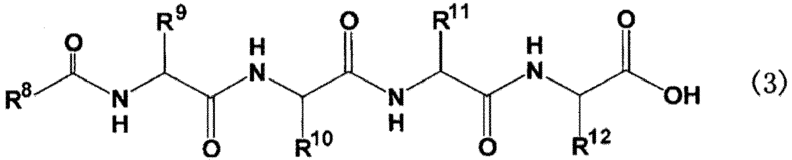 (dans laquelle R⁸ est un groupe aliphatique en C₉₋₂₃ ; et R⁹ à R¹² sont chacun indépendamment un atome d'hydrogène, un groupe alkyle en C₁₋₄ présentant, facultativement, une chaîne ramifiée en C₁ ou C₂ ou un groupe -(CH₂)ₙ-X, dans laquelle n est un nombre de 1 à 4 et X est un groupe amino, un groupe guanidino, un groupe -CONH₂ ou un cycle à 5 chaînons ou un cycle à 6 chaînons présentant, facultativement, un à trois atomes d'azote ou un cycle hétérocyclique condensé composé du cycle à 5 chaînons et du cycle à 6 chaînons) ; ou
des sels pharmaceutiquement utilisables de ceux-ci ;
une étape de préparation consistant à ajouter au moins un alcool monohydrique saturé ou insaturé présentant un nombre d'atomes de carbone de 8 à 30 au système de mélange chauffé pour préparer la composition aqueuse selon l'une quelconque des revendications 12 à 17 ;
une étape de mélange consistant à ajouter la composition aqueuse préparée à une phase aqueuse chauffée à une température n'étant pas inférieure à la température ambiante et inférieure à 100°C, suivie par un mélange, ou à ajouter une phase aqueuse chauffée à une température n'étant pas inférieure à la température ambiante et inférieure à 100°C à la composition aqueuse préparée, suivie par un mélange ; et
une étape de refroidissement consistant à refroidir le mélange obtenu à l'étape de mélange pour former un gel.

12. Procédé selon la revendication 11, dans lequel, à l'étape de mélange, une solution d'un médicament est en outre ajoutée.
